(19) Europäisches Patentamt
European Patent Office
Office européen des brevets

(11) EP 4 792 821 A2

(12) EUROPEAN PATENT APPLICATION

(43) Date of publication:
**19.08.2026 Bulletin 2026/34**

(21) Application number: **25224661.6**

(22) Date of filing: **17.12.2025**

(51) International Patent Classification (IPC):
***A61K 38/00*** *(2006.01)* ***C07K 7/08*** *(2006.01)*
***C07K 14/005*** *(2006.01)* ***C07K 7/06*** *(2006.01)*

(52) Cooperative Patent Classification (CPC):
**C07K 14/005;** A61K 38/00

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **14.02.2025 CN 202510165137**
**17.09.2025 CN 202511328719**

(71) Applicant: **Beijing Youcare Kechuang Pharmaceutical Technology Co., Ltd.**
**Beijing 100176 (CN)**

(72) Inventors:
- **SONG, Gengshen**
  **Beijing, 100176 (CN)**
- **ZHANG, Man**
  **Beijing, 100176 (CN)**
- **LIU, Zhaoguo**
  **Beijing, 100176 (CN)**
- **YU, Fei**
  **Beijing, 100176 (CN)**
- **ZHANG, Yingxin**
  **Beijing, 100176 (CN)**
- **LI, Jing**
  **Beijing, 100176 (CN)**
- **CUI, Yiwen**
  **Beijing, 100176 (CN)**
- **WANG, Donghang**
  **Beijing, 100176 (CN)**
- **YANG, Wei**
  **Beijing, 100176 (CN)**
- **XIE, Chao**
  **Beijing, 100176 (CN)**

(74) Representative: **Novagraaf Technologies**
**2 rue Sarah Bernhardt**
**CS90017**
**92665 Asnières-sur-Seine Cedex (FR)**

Remarks:
The complete document including Reference Table(s) and the Sequence Listing(s) can be downloaded from the EPO website

# (54) ANTIVIRAL POLYPEPTIDE MEMBRANE FUSION INHIBITOR, AND PREPARATION METHOD AND APPLICATION THEREOF

(57) The present disclosure relates to a polypeptide membrane fusion inhibitor against *Paramyxoviridae* and/or *Pneumoviridae* viruses, and a preparation method and application thereof. Provided in the present disclosure are a compound, a pharmaceutically acceptable salt or derivative thereof, a pharmaceutical composition and formulation product containing same, and applications thereof for preparation of drugs for preventing and/or treating diseases caused by *Paramyxoviridae* and/or *Pneumoviridae* viruses.

EP 4 792 821 A2

# Description

## Technical Field

The present disclosure belongs to the technical field of biomedicine, and relates to an antiviral polypeptide membrane fusion inhibitor, and a preparation method and application thereof, specifically relating to a type of polypeptide, especially a type of polypeptide against *Paramyxoviridae* and/or *Pneumoviridae* viruses, as well as an application of the polypeptide in preparation of virus membrane fusion inhibitors and preparation of drugs for treating or preventing infections caused by *Paramyxoviridae* and/or *Pneumoviridae* viruses.

## Background

*Paramyxoviridae* and *Pneumoviridae* viruses are two types of negative-sense single-stranded RNA viruses that pose serious threats to human and animal health. The diseases caused by these viruses are characterized by high pathogenicity and high infectivity, and there are currently no specific treatments available, making them major challenges in global public health.

In *Paramyxoviridae,* the Measles virus may cause acute respiratory infections and systemic complications in children, and localized outbreaks remain possible even in areas with widespread vaccination coverage. Mumps virus-induced non-purulent swelling of the parotid gland is often accompanied by serious complications such as orchitis, meningoencephalitis, etc. Emerging viruses such as *Nipahvirus, Hendravirus* show extremely high fatality rates and possess human-to-human transmission potential, which are classified as Biosafety Level 4 (BSL-4) pathogens. In the *Pneumoviridae,* human Respiratory Syncytial Virus (hRSV) is a primary pathogen causing lower respiratory tract infections (such as bronchiolitis and pneumonia) in infants and young children, resulting in millions of pediatric hospitalizations worldwide each year, and there are no specific therapeutic drugs available. Human metapneumovirus (hMPV) causes acute respiratory illness across all age groups, posing a significant threat particularly to the elderly and immunocompromised individuals.

At present, clinical interventions against the above viruses show significant limitations. Existing vaccines are only effective against a few viruses such as measles and mumps, and cannot cover all prevalent subtypes. Commonly used antiviral drugs have limited efficacy and are associated with significant toxic side effects (such as hemolytic anemia and teratogenicity), etc., and are largely ineffective against most viruses of *Paramyxoviridae* and *Pneumoviridae* families. Although antibody-based drugs offer some preventive effect against hRSV, the antibody drugs are expensive, complex administration routes, and can induce drug-resistant mutations in the viruses. Furthermore, the genomes of both virus types are prone to mutation, leading to a persistent decline in the effectiveness of existing interventions and further exacerbating treatment challenges.

In this context, the development of novel antiviral agents with broad-spectrum antiviral activity, low toxicity and side effects, and reduced susceptibility to inducing drug resistance has become an urgent priority. Due to small molecular weight, well-defined mechanisms of action, and excellent biocompatibility, antiviral polypeptides have gradually emerged as a key direction in antiviral drug development. However, study on specific antiviral peptides targeting *Paramyxoviridae* and *Pneumoviridae* viruses remains in the exploratory phase, with no mature products yet available for clinical treatment. Therefore, there is an urgent need to develop novel antiviral polypeptides capable of efficiently inhibiting infections caused by both types of viruses, so as to fill the gap in clinical treatment and deal with the public health threats caused by related viruses.

## Summary

In order to solve the defects in the related art, the present disclosure provides a membrane fusion inhibitory polypeptide.

Based on the previous research of the inventor, the present disclosure uses a polypeptide template of a sequence (1) (SEQ ID NO:103):

(1) $Z_1$-WJJLVOOSJJFDOOIJJVNOOIJJSLOOIJJSDOOLJJVNOOLJJTNOOITTI-$Z_3$-$Z_2$

In the template, $Z_1$ is an amino terminal ($NH_2$-) or a modification thereof; and $Z_2$ is a carboxyl terminal (-COOH) or a modification thereof. Unless otherwise specified, $Z_1$ is selected as an acetyl group (Ac-), and $Z_2$ is selected as an amido group (-$NH_2$), so as to respectively cap an amino terminal and carboxyl terminal of a polypeptide, thereby improving the stability of the polypeptide.

J is an acidic amino acid residue, which may be, but is not limited to, a glutamic acid residue and an aspartic acid residue. O is an alkaline amino acid residue, which may be, but is not limited to, a lysine residue and an arginine residue; a salt bridge is formed between J and O, and plays a universal role in stabilizing a secondary structure of the polypeptide; the remaining positions are specific amino acid residues, and the residues occupy corresponding positions of CHR natural

sequence, and a hydrophobic surface is formed mainly based on a hydrophobic amino acid residue, and in contact with and matches a drug target, thereby generating specific interactions. $Z_3$ is a fatty acid modifying group, including a linker arm and a lipophilic group, where the lipophilic group binds to a target cell of a drug.

A method for training a peptide-receptor binding activity prediction model is further used to design a specific polypeptide.

The present disclosure solves the above technical problems through the following technical solutions.

The method for training a peptide-receptor binding activity prediction model is further used to design a polypeptide compound of the present disclosure.

In an aspect, the present disclosure provides a compound, and a pharmaceutically acceptable salt or derivative thereof. The compound, the pharmaceutically acceptable salt or derivative thereof includes polypeptides that are selected from one or more of the following sequences shown in Formula I, Formula II, Formula III, Formula IV, SEQ ID NO:57-59, SEQ ID NO:82-88, and SEQ ID NO:97-102.

In another aspect, the present disclosure further provides use of the compound, the pharmaceutically acceptable salt or derivative thereof in preparation of a polypeptide membrane fusion inhibitor against *Paramyxoviridae* and/or *Pneumoviridae* viruses.

In another aspect, the present disclosure further provides an isolated nucleic acid molecule, which encodes the compound, the pharmaceutically acceptable salt or derivative thereof.

In another aspect, the present disclosure further provides a recombinant vector, which includes the nucleic acid molecule.

In another aspect, the present disclosure further provides a recombinant cell, which includes the nucleic acid molecule or the recombinant vector.

In another aspect, the present disclosure further provides a pharmaceutical composition, which includes the compound, the pharmaceutically acceptable salt or derivative thereof, the nucleic acid molecule, the recombinant vector, or the recombinant cell.

In another aspect, the present disclosure further provides a formulation product, which includes the pharmaceutical composition.

In another aspect, the present disclosure further provides a method for preparing the compound, the pharmaceutically acceptable salt or derivative thereof. The preparation method includes: a carrier resin is used, and protecting amino acids corresponding to a polypeptide amino acid sequence are coupled through deprotection and coupling reactions, so as to prepare a salt-bridge naked peptide.

In another aspect, the present disclosure further provides use of the compound, the pharmaceutically acceptable salt or derivative thereof, the pharmaceutical composition, or the formulation product in preparation of drugs for preventing and/or treating diseases caused by *Paramyxoviridae* and/or *Pneumoviridae* viruses.

In another aspect, the present disclosure further provides a method for preventing and/or treating diseases caused by *Paramyxoviridae* and/or *Pneumoviridae* viruses. The method includes: an effective dosage of the compound, the pharmaceutically acceptable salt or derivative thereof, the pharmaceutical composition, or the formulation product is applied to a subject in need.

In another aspect, the present disclosure further provides the compound, the pharmaceutically acceptable salt or derivative thereof, the pharmaceutical composition, or the formulation product, which is used for preventing and/or treating diseases caused by *Paramyxoviridae* and/or *Pneumoviridae* viruses.

In another aspect, the present disclosure further provides a method for inhibiting *Paramyxoviridae* and/or *Pneumoviridae* viruses. The method includes: the compound, the pharmaceutically acceptable salt or derivative thereof, the pharmaceutical composition, or the formulation product is applied to a sample.

On the basis of conforming to common knowledge in the field, the foregoing preferred conditions may be combined arbitrarily to obtain preferred embodiments of the present disclosure.

The present disclosure has the following beneficial effects.

The polypeptide designed in the present disclosure can effectively inhibit infections caused by RSV, hMPV, and human Parainfluenza Virus (hPIV).

## Detailed Description of the Embodiments

### Definitions

In order to understand the present disclosure more easily, some terms are defined first. Furthermore, it is to be noted that, whenever a value or parameter range is enumerated, the purpose is to indicate that intermediate values and ranges of these reference values also form part of the present disclosure.

The articles "a" and "an" used here refer to one or more (i.e., at least one) grammatical objects of the article. For example, "an element" refers to one element or more than one element, such as a plurality of elements.

The term "including" as used herein means a phrase "including but not limited to" and is interchangeable with it.

The term "or" as used herein refers to the term "and/or" and is interchangeable with it, unless the context clearly indicates otherwise.

The abbreviations used in the present disclosure have the following meanings:

Alanine (Ala, A)

Arginine (Arg, R)

Asparagine (Asn, N)

Asparticacid (Asp, D)

Dichloromethane (DCM)

N,N-Dimethyl malonate (DMF)

Envelope glycoprotein (Env)

Electronic Spray Ion Mass Spectroscopy (ESI-MS)

Fluorenylmethoxycarbonyl (Fmoc)

Glycine (Gly, G)

Glutamine (Gln, Q)

Glutamic acid (Glu, E)

six-helix bundle (6-HB)

2-(1H-1-hydroxybenzotriazole)-1,1,3,3-tetramethyl hexafluorophosphoric acid (HBTU)

Histidine (His, H)

1-Hydroxyl benzotiazole anhydrous (HoBt)

N-terminal heptad repeat (NHR)

C-terminal heptad repeat (CHR)

Human Immunodeficiency Virus (HIV)

High Performance Liquid Chromatography (HPLC)

Isoleucine (Ile, I)

Leucine (Leu, L)

Methionine (Met, M)

Lysine (Lys, K)

Phenylalanine (Phe, F)

Respiratory Syncytial Virus (RSV)

human Parainfluenza Virus (hPIV)

human metapneumovirus (hMPV)

Serine (Ser, S)

Trifluoroacetic acid (TFA)

Threonie (Thr, T)

Tyrosine (Tyr, Y)

Valine (Val, V)

Single-letter amino acid residues are represented as follows:
A: L-alanine; C: L-cysteine; D: L-aspartic acid; E: L-glutamic acid; F: L-phenylalanine; G: L-glycine; H: L-histidine; I: L-Isoleucine; K: L-lysine; L: L-leucine; M: L-methionine; N: L-aspartic acid; P: L-proline; Q: L-glutamine; R: L-arginine; S: L-serine; T: L-threonine; V: L-valine; W: L-tryptophan; and Y: L-tyrosine.

In an aspect, the present disclosure provides a compound, and a pharmaceutically acceptable salt or derivative thereof. The compound, the pharmaceutically acceptable salt or derivative thereof include polypeptides that are selected from one or more of the following sequences shown in Formula I, Formula II, Formula III, Formula IV, SEQ ID NO:57-59, SEQ ID NO:82-88, and SEQ ID NO:97-102.

Formula I (SEQ ID NO:122):

$X_1$-$X_2$-$X_3$-$X_4$-$X_5$-$X_6$-$X_7$-$X_8$-$X_9$-I-$X_{11}$-E-$X_{13}$-$X_{14}$-$X_{15}$-$X_{16}$-IEE-$X_{20}$-L-$X_{22}$-$X_{23}$-$X_{24}$-$X_{25}$-ESD-$X_{29}$-$X_{30}$-L-$X_{32}$-$X_{33}$-$X_{34}$-$X_{35}$-$X_{36}$-$X_{37}$-$X_{38}$.

wherein, $X_1$ is D or absent; $X_2$ is E or absent; $X_3$ is W, F, D, S, or absent; $X_4$ is D or absent; $X_5$ is E, A, K, or absent; $X_6$ is F, S, or absent; $X_7$ is D, N, L, or absent; $X_8$ is K, Q, L, or absent; $X_9$ is K or absent; $X_{11}$ is E or N; $X_{13}$ is V or E; $X_{14}$ is N or K; $X_{15}$ is K, R, or E; $X_{16}$ is K or R; $X_{20}$ is S, I, or L; $X_{22}$ is K or R; $X_{23}$ is K or R; $X_{24}$ is I or H; $X_{25}$ is E or N; $X_{29}$ is K or R; $X_{30}$ is K or R; $X_{32}$ is E or absent; $X_{33}$ is E, V, or absent; $X_{34}$ is V, N, S, or absent; $X_{35}$ is N, K, D, or absent; $X_{36}$ is K or absent; $X_{37}$ is K, A, or absent; and $X_{38}$ is L, A, or absent.

Formula II (SEQ ID NO:123):

$X'_1$-$X'_2$-$X'_3$-$X'_4$-$X'_5$-$X'_6$-E-$X'_8$-$X'_9$-$X'_{10}$-$X'_{11}$-$X'_{12}$-$X'_{13}$-$X'_{14}$-SQVNEKIN-$X'_{23}$-SL-$X'_{26}$-$X'_{27}$-IR-$X'_{30}$-$X'_{31}$-$X'_{32}$-$X'_{33}$-$X'_{34}$-KSDELL-$X'_{41}$-$X'_{42}$-$X'_{43}$-$X'_{44}$-$X'_{45}$-$X'_{46}$-$X'_{47}$-$X'_{48}$-$X'_{49}$-$X'_{50}$;

wherein, $X'_1$ is F, W, D, L, or Y; $X'_2$ is V or absent; $X'_3$ is K or absent; $X'_4$ is D, K, or absent; $X'_5$ is F, I, or absent; $X'_6$ is D or absent; $X'_8$ is L or absent; $X'_9$ is V or absent; $X'_{10}$ is F or absent; $X'_{11}$ is E or D; $X'_{12}$ is A or I; $X'_{13}$ is S or absent; X'14 is I or absent; $X'_{23}$ is E or Q; $X'_{26}$ is A or E; $X'_{27}$ is F, E, or K; $X'_{30}$ is L or absent; $X'_{31}$ is A or absent; $X'_{32}$ is F or absent; $X'_{33}$ is I or absent; $X'_{34}$ is R or absent; $X'_{41}$ is H or absent; $X'_{42}$ is N or absent; $X'_{43}$ is V or absent; X'44 is N or absent; $X'_{45}$ is A or absent; $X'_{43}$ is G or absent; $X'_{47}$ is K, L, or absent; $X'_{48}$ is S or absent; $X'_{49}$ is T or absent; and $X'_{50}$ is T or absent.

Formula III (SEQ ID NO:124):

$X''_1$-$X''_2$-$X''_3$-$X''_4$-D-$X''_6$-$X''_7$-IEEVN-$X''_{13}$-$X''_{14}$-IEESL-$X''_{20}$-$X''_{21}$-IEESD-$X''_{27}$-$X''_{28}$-L-$X''_{30}$-$X''_{31}$-V-$X''_{33}$-$X''_{34}$;

wherein, $X''_1$ is W or F; $X''_2$ is D or absent; $X''_3$ is E or absent; $X''_4$ is F or absent; $X''_6$ is K or A; $X''_7$ is K or S; $X''_{13}$ is K or R; $X''_{14}$ is K or R; $X''_{20}$ is K or R; $X''_{21}$ is K or R; $X''_{27}$ is K or R; $X''_{28}$ is K or R; $X''_{30}$ is E or H; $X''_{31}$ is E or N; $X''_{33}$ is N or absent; $X''_{34}$ is A or absent.

Formula IV (SEQ ID NO:125):
WDEFDASISQ-$X'''_{11}$-NEKINQSLEEIRKSDELLHN-$X'''_{32}$-$X'''_{33}$-$X'''_{34}$-$X'''_{35}$;
wherein, $X'''_{11}$ is V or absent; $X'''_{32}$ is V, N, or absent; $X'''_{33}$ is N, A, or absent; $X'''_{34}$ is A, L, or absent; and $X'''_{35}$ is L or

absent.

In some preferred implementations, the modified polypeptide has the structure of $R_1$-XX-$R_2R_3$, where XX is the above polypeptide; $R_1$ is an amino-terminal protecting group; $R_2$ is absent or an arbitrarily-substituted linker arm; and $R_3$ is a carboxyl-terminal protecting group.

In some preferred implementations, the compound, the pharmaceutically acceptable salt or derivative thereof includes polypeptides that are selected from one or more of sequences shown in SEQ ID NO:52-102.

In the present disclosure, the polypeptide defined as above is a core sequence included in the compound, the pharmaceutically acceptable salt or derivative thereof. The core sequence is the key component that mediates an inhibitory effect, which can act on an NHR region of an F protein, form an inactive 6-helix structure with the NHR, or disrupt an NHR structure to inhibit a key step of viral infection, that is, a process of forming a 6-helix bundle between NHR and CHR in the F protein, thereby preventing viruses from fusing with target cells. The inhibitory effect does not depend on modifications at both ends of the polypeptide, a linker arm, or modification by a lipophilic compound.

In some preferred implementations, the compound, the pharmaceutically acceptable salt or derivative thereof includes polypeptides that are selected from one or more of the following sequences shown in Formula I-1, Formula II-1, Formula III-1, Formula IV-1, and SEQ ID NO:106-121:

Formula I-1 (SEQ ID NO:126):

$R_1$-$X_1$-$X_2$-$X_3$-$X_4$-$X_5$-$X_6$-$X_7$-$X_8$-$X_9$-I-$X_{11}$-E-$X_{13}$-$X_{14}$-$X_{15}$-$X_{16}$-IEE-$X_{20}$-L-$X_{22}$-$X_{23}$-$X_{24}$-$X_{25}$-ESD-$X_{29}$-$X_{30}$-L-$X_{32}$-$X_{33}$-$X_{34}$-$X_{35}$-$X_{36}$-$X_{37}$-$X_{38}$-$R_2$-$R_3$;

Formula II-1 (SEQ ID NO:127):

$R_1$-$X'_1$-$X'_2$-$X'_3$-$X'_4$-$X'_5$-$X'_6$-E-$X'_8$-$X'_9$-I-$X'_{11}$-$X'_{12}$-$X'_{13}$-$X'_{14}$-SQVNEKIN-$X'_{23}$-SL-$X'_{26}$-$X'_{27}$-IR-$X'_{30}$-$X'_{31}$-$X'_{32}$-$X'_{33}$-$X'_{34}$-KSDELL-$X'_{41}$-$X'_{42}$-$X'_{43}$-$X'_{44}$-$X'_{45}$-$X'_{46}$-$X'_{47}$-$X'_{48}$-$X'_{49}$-$X'_{50}$-$R_2$-$R_3$;

Formula III-1 (SEQ ID NO:128):

$R_1$-$X''_1$-$X''_2$-$X''_3$-$X''_4$-D-$X''_6$-$X''_7$-IEEVN-$X''_{13}$-$X''_{14}$-IEESL-$X''_{20}$-$X''_{21}$-IEESD-$X''_{27}$-$X''_{28}$-L-$X''_{30}$-$X''_{31}$-V-$X''_{33}$-$X''_{34}$-$R_2$-$R_3$;

Formula IV-1 (SEQ ID NO:129):
R1-WDEFDASISQ-$X'''_{11}$-NEKINQSLEEIRKSDELLHN-$X'''_{32}$-$X'''_{33}$-$X'''_{34}$-$X'''_{35}$-$R_2$-$R_3$;

SEQ ID NO:106: R1-ADAFRLEVNDASSKINESIEESLLSLEKLHNVNATA-$R_2$-$R_3$;

SEQ ID NO:107: R1-FDAFIQEINVNEDQSLEQSDELLLELHLLHSLLH-$R_2$-$R_3$;

SEQ ID NO:108: R1-FAEFNQKINQVNEKIEESLEEIRKSDEELHNVNATT-$R_2$-$R_3$;

SEQ ID NO:109: R1-SDEQVNEKINQSLAFIRRIRKLLHN-$R_2$-$R_3$;

SEQ ID NO:110: R1-ILELVNKKIEQSLKFIEKSDKLLEN-$R_2$-$R_3$;

SEQ ID NO:111: R1-SLEQVNKKINQSLKVNKKSDKLLEN-$R_2$-$R_3$;

SEQ ID NO:112: R1-FDEEVNKKIEQSLKINQSLEEIRKS-$R_2$-$R_3$;

SEQ ID NO:113: R1-SISQVNEKINEIQSLEEKSDKLLKS-$R_2$-$R_3$;

SEQ ID NO:114: R1-VNKKIEEEKQSLKKQSDKIEESDEN-$R_2$-$R_3$;

SEQ ID NO:115: R1-FDELVNKKIEKIEEVNKKSLKLLES-$R_2$-$R_3$ ;

SEQ ID NO:116: R1-FEVNRRRIEQSLEKSLESLEEEEHSDKKLHNELH-$R_2$-$R_3$;

SEQ ID NO:117: R1-SLEQVNKINKKIDKIEESLKKIEESDKKSLEVNKKL-$R_2$-$R_3$;

SEQ ID NO:118: R1-SLEQVNKINEKINKISQSLKKIEESDKKSDEVNAGL-$R_2$-$R_3$;

SEQ ID NO:119: R1-SLEQVNKKIEQSLESLKKSDKINQSLEEVNKSDELL-$R_2$-$R_3$;

SEQ ID NO:120: R1-SLEQVNEKINQSLAFIRKSDEINQSDEEVNKSDELL-$R_2$-$R_3$; and

SEQ ID NO:121: R1-SDELVNKKIEFDKKINQSLKKIEESDKKKL-$R_2$-$R_3$.

$R_1$ is an amino-terminal protecting group, preferably acetyl.

$R_2$ is absent or an arbitrarily-substituted linker arm, preferably -$R_4$-$R_5$($R_6$)-, where $R_4$ is a polypeptide, of which amino acid sequence is preferably $(EAAAK)_m$ or $(GSGSG)_m$; m is a natural number from 0 to 5; and specifically, m may be any natural number from 0, 1, 2, 3, 4, and 5.

$R_5$ is lysine, cysteine, 2,3-diaminopropionic acid, ornithine, 2,4-diamino-butyric acid, or 2,7-diaminoheptanoic acid, preferably, the lysine.

$R_6$ is a lipophilic compound group that is modified on R5. Preferably, the lipophilic compound group is selected from one or more of cholesterol, cholesteryl hemisuccinate, 2-cholesterol acetic acid, 2-cholesterol propionic acid, 3-cholesterol propionic acid, 2-cholesterol butyric acid, 2-cholesterol isobutyric acid, 3-cholesterol butyric acid, 3-cholesterol isobutyric acid, 4-cholesterol butyric acid, 2-cholesterol valeric acid, 2-cholesterol isovaleric acid, 3-cholesterol valeric acid, 5-cholesterol valeric acid, 2-cholesterol hexanoic acid, 6-cholesterol hexanoic acid, 2-cholesterol heptanoic acid, 7-cholesterol heptanoic acid, 2-cholesterol octanoic acid, 8-cholesterol octanoic acid, bromoacetate cholesterol ester, cholesteryl chloroformate, palmitic acid, stearic acid, fatty acid containing 3-20 carbon atoms, binary fatty acid containing 3-20 carbon atoms, and other groups that can act with a cell membrane or viral envelope to improve the effect of the polypeptide and the cell membrane or viral envelope, more preferably, the cholesteryl hemisuccinate.

$R_3$ is a carboxyl-terminal protecting group, preferably -$NH_2$.

In some preferred implementations, the compound, the pharmaceutically acceptable salt or derivative thereof includes polypeptides that are selected from one or more of sequences shown in SEQ ID NO:1-51.

In some implementations, the derivative is a solvate, a chelate, or a non-covalent complex.

In some implementations, the pharmaceutically acceptable salt includes: acetate, lactobionate, benzene sulfonate, laurate ester, benzoate, malate, bicarbonate, maleate, bisulfate, mandelate, bitartrate, mesylate, borate, methyl bromide, bromide, methyl nitrate, calcium edetate, methylsulfate, d-camphorsulfonic acid, acetate, carbonate, naphthalenesulfonate, chloride, nitrate, benzoate, n-methylglucosamine, citrate, ammonium salt, dihydrochloride, oleate, ethylenediaminetetraacetic acid salt, oxalate, edisylate, pamoate, pamoate, lauryl propionate and lauryl sulfate, palmitate, ethanesulfonate, pantothenate, fumarate, phosphate/diphosphate, glucoheptonate, polygalacturonate, gluconate, salicylate, glutamate, stearate, hydroxyacetyl aminophenylarsonic acid, sulfate, hydroxybenzoate, basic acetate, succinate, hydrobromide, tannate, hydrochloride, tartrate, hydroxy naphthate, 8-chlorotheophylline salt, iodide, toluenesulfonate, triethyl iodide, lactic acid, valerate, etc. Depending on the application, the pharmaceutically acceptable salt may be formed by cations such as sodium, potassium, or bismuth, etc., or by bases such as ammonia, ethylenediamine, N-methyl-L-glutamine, lysine, arginine, ornithine, choline, N,N'-Dibenzylethylenediamine, chloroprocain, diethanol amine, procaine, diethylamine, piperazine, tris(hydroxymethyl)aminomethane, and tetramethylammonium hydroxide, etc. These salts may be prepared by standard methods, for example, by reacting the free acids with organic or inorganic bases. In a case where an basic group such as an amino group is presented, acid salts such as hydrochloride, hydrobromide, acetate, pamoate, etc. may be used as pharmaceutical forms. In a case where an acidic group or an alcohol group is presented, pharmaceutically acceptable esters such as acetate, maleate, chloromethyl trimethylacetate, etc., as well as other esters known in the literature for improving solubility and hydrolysis may be used as sustained-release formulations or prodrugs.

In another aspect, the present disclosure provides the use of the compound, the pharmaceutically acceptable salt or derivative thereof as disclosed herein in preparation of a polypeptide membrane fusion inhibitor against *Paramyxoviridae* and/or *Pneumoviridae* viruses.

Herein, the "polypeptide membrane fusion inhibitor against *Paramyxoviridae* and/or *Pneumoviridae* viruses" is a specially designed polypeptide drug, which can inhibit fusion of a viral envelope and a host cell membrane during infections caused by *Paramyxoviridae* and/or *Pneumoviridae* viruses. By blocking this key step, viruses cannot enter host cells, thereby preventing viral replication and transmission.

In another aspect, the present disclosure provides an isolated nucleic acid molecule. The nucleic acid molecule encodes the compound, the pharmaceutically acceptable salt or derivative thereof as disclosed herein.

In another aspect, the present disclosure provides a recombinant vector. The recombinant vector includes the

nucleic acid molecule as disclosed herein.

In another aspect, the present disclosure provides a recombinant cell. The recombinant cell includes the nucleic acid molecule as disclosed herein or the recombinant vector as disclosed herein.

In another aspect, the present disclosure provides a pharmaceutical composition. The pharmaceutical composition includes the compound, the pharmaceutically acceptable salt or derivative thereof as disclosed herein, the nucleic acid molecule as disclosed herein, the recombinant vector as disclosed herein, or the recombinant cell as disclosed herein; optionally, further includes a pharmaceutically acceptable carrier or excipient.

The pharmaceutical composition of the present disclosure may be a solution with or without a buffer, or a composition containing a pharmaceutically acceptable carrier. In the present disclosure, the pharmaceutical composition may be administered in a solution, and may be administered in a non-buffered solution such as normal saline or water. Alternatively, the pharmaceutical composition may also be administered in a suitable buffer solution. The buffer solution may include acetate, citrate, prolamin, carbonate, or phosphate, or any combination thereof. In a preferred embodiment, the buffer solution is Phosphate Buffered Saline (PBS). The pH and osmolarity of the buffer of the pharmaceutical composition may be adjusted to values suitable for administration to a subject.

In some embodiments, the buffer solution further includes a reagent for controlling the osmolality of the solution, such that the osmolality is maintained at an expected value, for example, a physiological value of human plasma. Solutes that may be added to the buffer solution to control the osmolality include (but are not limited to) proteins, peptides, amino acids, non-metabolic polymers, vitamins, ions, sugar, metabolites, organic acids, lipids, or salts. In some embodiments, the reagent for controlling the osmolality of the solution is a salt. In some embodiments, the reagent for controlling the osmolality of the solution is sodium chloride or potassium chloride.

The pharmaceutical composition of the present disclosure may be administered at a dosage sufficient to inhibit virus. Generally, the suitable dosage of the compound of the present disclosure for use in mammals, particularly humans, may range from 0.1 mg/day to 100 mg/day, for example, from 10 mg/day to 50 mg/day, or for another example, from 20 mg/day to 30 mg/day.

The pharmaceutical composition may be administered once daily, or may be administered in two, three, or more sub-dosages at appropriate intervals throughout the day, or may even be administered via continuous infusion or delivery using a controlled-release formulation. In these cases, the compound contained in each sub-dosage must be less, so as to achieve a total daily dosage. A dosage unit may also be combined for delivery over several days, for example, using conventional sustained-release formulations that provide a sustained release of the compound over a period of days. The sustained-release formulations are well known in the art and are particularly useful for delivering agents at specific sites, and thus may be used with the agents of the present disclosure. In this embodiment, the dosage unit includes a plurality of corresponding daily dosages.

In other embodiments, a single dosage of the pharmaceutical composition may be long-lasting and sustained, allowing subsequent dosages to be administered at intervals of no more than 3, 4, or 5 days, or at intervals of no more than 1, 2, 3, or 4 weeks. A treatment regimen may include administration once every 1-3 days for a continuous period of 4-7 days. If the infection recurs, another 4-7 days of continuous administration may be performed, and a treatment cycle for administration may range from 1 to 7. In some embodiments of the present disclosure, a single dosage of the pharmaceutical composition of the present disclosure is administered once weekly. In other embodiments of the present disclosure, a single dosage of the pharmaceutical composition of the present disclosure is administered once monthly.

Those skilled in the art will understand that certain factors may influence the dosage and time arrangement required for effective treatment of a subject. These factors include (but are not limited to) the severity of the disease or condition, prior treatments, the overall health and/or age of the subject, and other existing diseases. Furthermore, the administration of a therapeutically effective dosage of the composition to a subject may include a single treatment or a series of treatments. As described elsewhere herein, the effective dosage and in-vivo half-life of each compound covered by the present disclosure may be estimated using conventional methods or in vivo tests based on the use of appropriate animal models.

Depending on whether topical or systemic treatment is desired and depending on the region to be treated, the pharmaceutical composition of the present disclosure may be administered in numerous ways. Administration may be topical (e.g., via transdermal patches); pulmonary, for example, via inhalation or blowing of powder or aerosols, including a sprayer; intratracheal; intranasal; and epidermal, transdermal, oral, or parenteral. Parenteral administration includes intravenous, intra-arterial, subcutaneous, intraperitoneal, or intramuscular injection or infusion; subcutaneous administration, for example, via an implantable device; or intracranial administration, for example, administration within the brain parenchyma, sheath, or heart ventricle.

The pharmaceutical composition of the present disclosure (which may conveniently be provided in the unit of a dosage form) may be prepared according to conventional techniques well known in the pharmaceutical industry. Such techniques include the following steps: these active ingredients are combined with the drug carrier or excipient. In general, these pharmaceutical compositions are prepared by the following steps: these active ingredients are uniformly and finely combined with a liquid carrier or a finely-dispersed solid carrier, or both, and a product is subsequently shaped if required.

In another aspect, the present disclosure provides a formulation product. The formulation product includes the pharmaceutical composition of the present disclosure.

Certain formulation products of the present disclosure also introduce a carrier material in the pharmaceutical composition. The carrier material includes, but is not limited to, a water-soluble carrier material (e.g., polyethylene glycol, polyvinylpyrrolidone, organic acid, etc.), an insoluble carrier material (e.g., ethyl cellulose, cholesteryl stearate, etc.), and an enteric carrier material (e.g., cellulose acetate phthalate, carboxymethyl cellulose, etc., preferably the water-soluble carrier material. Various formulation products may be prepared by using these materials, including but not limited to tablets, capsules, dropping pills, aerosols, pills, powders, solutions, suspensions, emulsions, granules, liposomes, transdermal agents, buccal tablets, suppositories, freeze-dried powder injections, etc. The formulation products may be conventional formulations, sustained-release formulations, controlled-release formulations, and various microparticle delivery systems. In order to prepare the unit dosage into tablets, various carriers well known in the art may be widely used. Examples of carriers include: diluents and absorbents such as starch, dextrin, calcium sulfate, lactose, mannitol, sucrose, sodium chloride, trehalose, glucose, urea, calcium carbonate, white clay, microcrystalline cellulose, aluminum silicate, etc.; humectants and binders, such as water, glycerin, polyethylene glycol, ethanol, propanol, starch slurry, dextrin, syrup, honey, glucose solutions, gum arabic slurry, gelatin slurry, sodium carboxymethyl cellulose, shellac, methyl cellulose, potassium phosphate, polyvinylpyrrolidone, etc.; disintegrating agents, such as dried starch, alginate, agar powder, brown algae starch, sodium bicarbonate, citric acid, calcium carbonate, polyoxyethylene, sorbitan fatty acid esters, sodium dodecyl sulfate, methyl cellulose, ethyl cellulose, etc.; disintegration inhibitors, such as sucrose, glyceryl tristearate, cocoa butter, hydrogenated oil, etc.; absorption enhancers, such as quaternary ammonium salts, sodium lauryl sulfate, etc.; and lubricants, such as talc, silica, cornstarch, stearates, boric acid, liquid paraffin, polyethylene glycol, etc. The tablets may further be prepared into coated tablets, such as sugar-coated tablets, film-coated tablets, enteric-coated tablets, or double-layer and multi-layer tablets. In order to prepare the unit dosage into pills, various carriers well known in the art may be widely used. Examples of carriers include: diluents and absorbents, such as glucose, lactose, starch, cocoa butter, hydrogenated vegetable oil, polyvinylpyrrolidone, Gelucire, kaolin, talc, etc.; adhesives, such as gum arabic, gum yarrow, gelatin, ethanol, honey, liquid sugar, rice paste, or flour paste, etc.; and dispersing agents, such as agar powder, dried starch, alginate, sodium dodecyl sulfate, methyl cellulose, ethyl cellulose, etc. In order to prepare the unit dosage into suppositories, various carriers well known in the art may be widely used. Examples of carriers include: polyethylene glycol, lecithin, cocoa butter, higher alcohols, esters of higher alcohols, gelatin, semi-synthetic glycerides, etc. In order to prepare the unit dosage into injection formulations, for example, solutions, emulsions, freeze-dried powder injections, and suspensions may use all diluents commonly used in the art, such as water, ethanol, polyethylene glycol, 1,3-propanediol, ethoxylated isostearyl alcohol, polyoxidized isostearyl alcohol, polyoxyethylene sorbitol fatty acid ester, etc. Furthermore, in order to prepare an isotonic injection solution, a proper amount of sodium chloride, glucose, or glycerol may be added to an injection formulation. Moreover, conventional cosolvents, buffer agents, pH regulators, etc. may also be added. Moreover, if necessary, colorants, preservatives, spice, flavoring agents, sweetening agents, or other materials may also be added to the drug formulation. The above dosage forms may be administered via injection, including subcutaneous injection, intravenous injection, intramuscular injection, intracavitary injection, etc.; may be administered via cavity channels such as rectum and vagina; may be administered via respiratory tracts such as the nasal cavity; and may be administered via mucous membranes. The above administration route is preferably injection.

In some implementations, the pharmaceutical composition of the present disclosure may be prepared as any one of a number of possible formulations. These formulations, for example, include, but are not limited to, any one of the following groups: an oral formulation, an injection formulation, an inhalation formulation, and a lyophilized preparation, preferably, the inhalant, and the lyophilized preparation, a subcutaneous injection, or an intramuscular injection.

In some implementations, an administration route of the formulation product is selected from any one of the following: oral administration, injection, mucosal administration, transdermal administration, and nebulization, preferably pulmonary nebulization, subcutaneous injection, or intramuscular injection.

In another aspect, the present disclosure provides a method for preparing the compound, the pharmaceutically acceptable salt or derivative thereof as disclosed herein. The method includes the following steps.

A carrier resin is used, and protecting amino acids corresponding to a polypeptide amino acid sequence are coupled through deprotection and coupling reactions, so as to prepare a salt-bridge naked peptide.

In some implementations, the method further includes one or more of the following steps.

(1) A lipophilic compound modification is performed, preferably, a cholesteryl hemisuccinate modification.

(2) N-terminal acetylation capping is performed.

(3) C-terminal amidation capping is performed.

In another aspect, the present disclosure provides use of the compound, the pharmaceutically acceptable salt or

derivative thereof of the present disclosure, the pharmaceutical composition as disclosed herein, or the formulation product as disclosed herein in preparation of drugs for preventing and/or treating diseases caused by *Paramyxoviridae* and/or *Pneumoviridae* viruses.

As used herein, "diseases caused by *Paramyxoviridae* and/or *Pneumoviridae* viruses" is intended to include any disease associated with infections caused by *Paramyxoviridae* and/or *Pneumoviridae* viruses.

In some implementations, *Paramyxoviridae* is selected from viruses of the genus *Respirovirus, Rubulavirus, Morbillivirus,* and *Henipavirus;* the *Pneumoviridae* is selected from viruses of the genus *Metapneumovirus* and *Orthopneumovirus.*

In some preferred implementations, the virus of the genus *Respirovirus* is a human parainfluenza virus (hPIV). The hPIV includes sub-types hPIV-1, hPIV-2, hPIV-3, and hPIV-4.

In some preferred implementations, the virus of the genus *Rubulavirus* is *Mumpsvirus.*

In some preferred implementations, the virus of the genus *Morbillivirus* is a measles virus.

In some preferred implementations, the virus of the genus *Henipavirus* is selected from *Nipahvirus* and *Hendravirus.*

In some preferred implementations, the virus of the genus *Metapneumovirus* is human metapneumovirus (hMPV).

In some preferred implementations, the virus of the genus *Orthopneumovirus* is Respiratory Syncytial Virus (RSV).

In some specific implementations, the hMPV is selected from hMPV type A1 (hMPV-A1), type A2 (hMPV-A2), type B1 (hMPV-B1), and type B2 (hMPV-B2).

In some preferred implementations, the RSV is selected from RSV-A, RSV-B, and a variant strain thereof.

In another aspect, the present disclosure provides a method for preventing and/or treating diseases caused by *Paramyxoviridae* and/or *Pneumoviridae* viruses. The method includes: an effective dosage of the compound, the pharmaceutically acceptable salt or derivative thereof as disclosed herein, the pharmaceutical composition of the present disclosure, or the formulation product of the present disclosure are applied to a subject in need.

As used herein, the "subject" is intended to include humans or non-human animals, preferably mammals such as mice. Most preferably, the subject or patient is human.

As used herein, "the effective dosage" is intended to include a dosage sufficient to achieve the treatment of a disease when administered to a patient for the treatment of the disease caused by *Paramyxoviridae* and/or *Pneumoviridae* viruses (e.g., by alleviating, improving, or maintaining the existing disease or one or more disease symptoms). The "effective dosage" may vary depending on how the agent is administered, the nature and severity of the disease, and the medical history, age, weight, family history, genetic composition, stage of the pathological process mediated by *Paramyxoviridae* and/or *Pneumoviridae* viruses, type of prior or concomitant therapy (if any), and other individual characteristics of a patient to be treated. The "effective dosage" further includes a dosage that produces a desired local or systemic effect within a rational benefit-risk ratio applicable to any treatment. The compound used in the method of the present disclosure may be administered in an amount sufficient to produce a rational benefit-risk ratio for such treatment.

In another aspect, the present disclosure provides the compound, the pharmaceutically acceptable salt or derivative thereof as disclosed herein, the pharmaceutical composition as disclosed herein, or the formulation product as disclosed herein, which is used for preventing and/or treating diseases caused by *Paramyxoviridae* and/or *Pneumoviridae* viruses.

In another aspect, the present disclosure provides a method for inhibiting *Paramyxoviridae* and/or *Pneumoviridae* viruses. The method includes: the compound, the pharmaceutically acceptable salt or derivative thereof of the present disclosure, the pharmaceutical composition of the present disclosure, or the formulation product of the present disclosure is applied to a sample.

As used herein, the term "sample" includes similar fluids, cells, or tissues isolated from the subject, as well as a collection of the fluids, cells, or tissues present in the subject. Examples of biological fluids include blood, serum, serosal fluid, plasma, cerebrospinal fluid, ocular fluid, lymph fluid, urine, saliva, etc. Tissue samples may include samples obtained from tissues, organs, or local areas. For example, the samples may be derived from specific organs, organ parts, or fluids or cells in these organs. In some embodiments, the "sample" refers to blood or plasma drawn from the subject.

The term "non-therapeutic purpose" here refers to the inhibition of replication of *Paramyxoviridae* and/or *Pneumoviridae* viruses for scientific research purposes, for example, in a laboratory.

The following embodiments are used to illustrate the present disclosure, but not to limit the scope of the present disclosure. If not specifically indicated, the technical means used in the embodiments are conventional means known to those skilled in the art, and raw materials used are commercially available.

### Embodiment 1: Polypeptide design

This disclosure designs polypeptides based on the CHR sequence of the F1 subunit of the F protein correspond-

ing to the virus. Firstly, a series of 36-mers covering the CHR region of the virus were designed, and the corresponding polypeptides were synthesized. Their activity was evaluated through molecular interaction experiments and antiviral activity assays, and further design and optimization of the polypeptides were conducted based on the test results. The inventors found that natural - sequence viral CHR polypeptides usually have the disadvantages of poor water solubility and difficulty in synthesis, which pose significant challenges to subsequent drug development. Drawing on the inventors' experience and previous research findings, this disclosure systematically introduces EE - KK salt bridges at the non - binding sites of the viral CHR polypeptide sequence to enhance its water solubility. Meanwhile, these salt bridges can stabilize the $\alpha$ - helical secondary structure required for the polypeptide to exert its activity. This disclosure reveals that the introduction of salt bridges not only improves water solubility but also significantly enhances the activity, enabling the design of shorter polypeptides.

Based on the above considerations, in the present invention, derivative polypeptides were prepared by introducing systematic mutations into Sequence (1) (SEQ ID NO: 103): $Z_1$-WJJLVOOSJJFDOOIJJVNOOIJJSLOOIJJS DOOLJJVNOOLJJTNOOITTI-$Z_3$ - $Z_2$. The modifications include mutating E and K in the salt bridges into the corresponding D and R, and simultaneously fine - tuning the binding sites to improve the binding affinity between the polypeptides and their targets, thereby completing the design of the polypeptides described in the examples.

Herein, $Z_1$ represents the amino - terminal ($NH_2$ -) or its modification; $Z_2$ represents the carboxyl - terminal (-COOH) or its modification; J denotes an acidic amino acid residue, including but not limited to glutamic acid residue and aspartic acid residue; O denotes a basic amino acid residue, including but not limited to lysine residue and arginine residue.

$Z_3$ is a fatty acid modification group, consisting of a linker arm and a lipophilic group. The lipophilic group binds to the target cells of the drug to enhance the drug activity, while the linker arm is used to connect the polypeptide chain and the lipophilic group. In addition, it provides appropriate steric space and conformation to facilitate better binding between the polypeptide drug and its target.

Table 1-1 Sequence design for polypeptide (Formula I-1)

| Compound | Sequence | SE Q ID NO : | Structure type |
|---|---|---|---|
| Compound 1 | Ac-FDASIEEVNRRIEESLRRIEESDRRLEVNKKA-$NH_2$ | 1 | Naked peptide |
| Compound 2 | Ac-DDEFNQKINEVNEKIEEILKKIEESDRKLEESDKKA-$NH_2$ | 2 | Naked peptide |
| Compound 3 | Ac-DEFDKKKIEEVNKKIEESLKKIEESDKKLEEV-$NH_2$ | 3 | Naked peptide |
| Compound 4 | Ac-FDASLLKIEEEKKKIEELLKLHNESDKKLEEV-$NH_2$ | 4 | Naked peptide |
| Compound 5 | Ac-WDEFDKKIEEVNKKIEESLKKIEESDKKLEEVNKKL-$NH_2$ | 5 | Naked peptide |
| Compound 9 | Ac-WDEFDKKIEEVNKKIEESLKKIEESDKKLEEVNKKL-EAAAKK(Chol)-$NH_2$ | 9 | Lipopeptide |
| Compound 10 | Ac-KIEEVNKKIEESLKKIEESDKKLEEVNKKL-EAAAKK(Chol)-$NH_2$ | 10 | Lipopeptide |
| Compound 11 | Ac-WDEFDKKIEEVNKKIEESLKKIEESDKKLEEVNKKL-GSGSGK(Chol)-$NH_2$ | 11 | Lipopeptide |
| Compound | Ac-FDASIEEVNRRIEESLRRIEESDRRLEVNKKA- | 12 | Lipopepti |
| 12 | GSGSGK(Chol)-$NH_2$ | | de |
| Compound 13 | Ac-WDEFDKKIEEVNKKIEESLKKIEESDKKL-EAAAKK(Chol)-$NH_2$ | 13 | Lipopeptide |

(continued)

| Compound | Sequence | SE Q ID NO : | Structure type |
|---|---|---|---|
| Compound 14 | Ac-DEFDKKIEEVNKKIEESLKKIEESDKKLEEVN-EAAAKK(Chol)-$NH_2$ | 14 | Lipopeptide |
| Compound 15 | Ac-DEFDKKIEEVNKKIEESLKKIEESDKKLEEV-GSGSGK(Chol)-$NH_2$ | 15 | Lipopeptide |
| Compound 16 | Ac-SDEFDKKIEEVNKKIEESLKKIEESDKKL-EAAAKK(Chol)-$NH_2$ | 16 | Lipopeptide |
| Compound 17 | Ac-DEFDKKIEEVNKKIEESLKKIEESDKKL-EAAAKK(Chol)-$NH_2$ | 17 | Lipopeptide |
| Compound 18 | Ac-DEFDKKIEEVNKKIEESLKKIEESDKKLEEV-GSGSGK(Chol)-$NH_2$ | 18 | Lipopeptide |
| Compound 19 | Ac-FDASIEEVNRRIEESLRRIEESDRRL-EAAAKK(Chol)-$NH_2$ | 19 | Lipopeptide |
| Compound 20 | Ac-SDEFDKKIEEVNKKIEESLKKIEESDKKL-GSGSGK(Chol)-$NH_2$ | 20 | Lipopeptide |
| Compound 21 | Ac-DEFDKKIEEVNKKIEESLKKIEESDKKLEEV-EAAAKK(Chol)-$NH_2$ | 21 | Lipopeptide |
| Compound 22 | Ac-DEFDKKIEEVNKKIEESLKKIEESDKKLEEV-EAAAKK-(Chol)-$NH_2$ | 22 | Lipopeptide |

Table 1-2 Sequence design for polypeptide (Formula II-1)

| Compound | Sequence | SEQ ID NO: | Structure type |
|---|---|---|---|
| Compound 23 | Ac-YDELVFDASQVNEKINQSLEKIRKSDELLHNV-$NH_2$ | 23 | Naked peptide |
| Compound 24 | Ac-FDFDEFDASISQVNEKINESLAFIRLAFIRKSDELL-EAAAKK-$NH_2$ | 24 | Naked peptide |
| Compound 25 | Ac-YDELVFDASQVNEKINQSLEKIRKSDELLHNV-GSGSGK(Chol)-$NH_2$ | 25 | Lipopeptide |
| Compound 26 | Ac-FDEEISQVNEKINQSLAFIRKSDELLHNVNAGKSTT-EAAAKK(Chol)-$NH_2$ | 26 | Lipopeptide |
| Compound 27 | Ac-WDEFDASISQVNEKINQSLEEIRKSDELLHNVNAGL-GSGSGK(Chol)-$NH_2$ | 27 | Lipopeptide |
| Compound 28 | Ac-DEFDASISQVNEKINQSLEEIRKSDELLHN-EAAAKK(Chol)-$NH_2$ | 28 | Lipopeptide |

(continued)

| Compound | Sequence | SEQ ID NO: | Structure type |
|---|---|---|---|
| Compound 29 | Ac-LVKKIDEFDASISQVNEKINQSLEEIRKSDELLHNV-GSGSGK(Chol)-$NH_2$ | 29 | Lipopeptide |
| Compound 30 | Ac-YDELVFDASQVNEKINQSLEKIRKSDELLHNV-EAAAKK(Chol)-$NH_2$ | 30 | Lipopeptide |

Table 1-3 Sequence design for polypeptide (Formula III-1)

| Compound | Sequence | SEQ ID NO: | Structure type |
|---|---|---|---|
| Compound 40 | Ac-WDEFDKKIEEVNKKIEESLKKIEESDKKLEEVNA-EAAAKK(Chol)-$NH_2$ | 40 | Lipopeptide |
| Compound 41 | Ac-WDEFDKKIEEVNKKIEESLKKIEESDKKLEEVN-EAAAKK(Chol)-$NH_2$ | 41 | Lipopeptide |
| Compound 42 | Ac-FDASIEEVNRRIEESLRRIEESDRRLEEVNA-EAAAKK(Chol)-$NH_2$ | 42 | Lipopeptide |
| Compound 43 | Ac-WDEFDASIEEVNRRIEESLRRIEESDRRLEEV-EAAAKK(Chol)-$NH_2$ | 43 | Lipopeptide |
| Compound 44 | Ac-WDEFDKKIEEVNKKIEESLKKIEESDKKLHNV-EAAAKK(Chol)-$NH_2$ | 44 | Lipopeptide |

Table 1-4 Sequence design for polypeptide (Formula IV-1)

| Compound | Sequence | SEQ ID NO: | Structure type |
|---|---|---|---|
| Compound 38 | Ac-WDEFDASISQVNEKINQSLEEIRKSDELLHNVNAL-EAAAKK(Chol)-$NH_2$ | 38 | Lipopeptide |
| Compound 39 | Ac-WDEFDASISQNEKINQSLEEIRKSDELLHNNAL-EAAAKK(Chol)-$NH_2$ | 39 | Lipopeptide |
| Compound 45 | Ac-WDEFDASISQVNEKINQSLEEIRKSDELLHN-EAAAKK(Chol)-$NH_2$ | 45 | Lipopeptide |

Table 1-5 Sequence design for polypeptide

| Compound | Sequence | SEQ ID NO: | Structure type |
|---|---|---|---|
| Compound 6 | Ac-ADAFRLEVNDASSKINESIEESLLSLEKLHNVNATA-$NH_2$ | 6 | Naked peptide |
| Compound 7 | Ac-FDAFIQEINVNEDQSLEQSDELLLELHLLHSLLH-$NH_2$ | 7 | Naked peptide |

(continued)

| Compound | Sequence | SEQ ID NO: | Structure type |
|---|---|---|---|
| Compound 8 | Ac-FAEFNQKINQVNEKIEESLEEIRKSDEELHNVNATT-$NH_2$ | 8 | Naked peptide |
| Compound 31 | Ac-SDEQVNEKINQSLAFIRRIRKLLHN-EAAAKK(Chol)-$NH_2$ | 31 | Lipopeptide |
| Compound 32 | Ac-ILELVNKKIEQSLKFIEKSDKLLEN-EAAAKK(Chol)-$NH_2$ | 32 | Lipopeptide |
| Compound 33 | Ac-SLEQVNKKINQSLKVNKKSDKLLEN-EAAAKK(Chol)-$NH_2$ | 33 | Lipopeptide |
| Compound 34 | Ac-FDEEVNKKIEQSLKINQSLEEIRKS-EAAAKK(Chol)-$NH_2$ | 34 | Lipopeptide |
| Compound 35 | Ac-SISQVNEKINEIQSLEEKSDKLLKS-EAAAKK(Chol)-$NH_2$ | 35 | Lipopeptide |
| Compound 36 | Ac-VNKKIEEEKQSLKKQSDKIEESDEN-EAAAKK(Chol)-$NH_2$ | 36 | Lipopeptide |
| Compound | Ac-FDELVNKKIEKIEEVNKKSLKLLES-EAAAKK(Chol)- | 37 | Lipopeptide |
| 37 | $NH_2$ | | de |
| Compound 46 | Ac-FEVNRRRIEQSLEKSLESLEEEEHSDKKLHNELH-$NH_2$ | 46 | Naked peptide |
| Compound 47 | Ac-SLEQVNKINKKIDKIEESLKKIEESDKKSLEVNKKL-EAAAKK-$NH_2$ | 47 | Naked peptide |
| Compound 48 | Ac-SLEQVNKINEKINKISQSLKKIEESDKKSDEVNAGL-EAAAKK-$NH_2$ | 48 | Naked peptide |
| Compound 49 | Ac-SLEQVNKKIEQSLESLKKSDKINQSLEEVNKSDELL-EAAAKK-$NH_2$ | 49 | Naked peptide |
| Compound 50 | Ac-SLEQVNEKINQSLAFIRKSDEINQSDEEVNKSDELL-EAAAKK-$NH_2$ | 50 | Naked peptide |
| Compound 51 | Ac-SDELVNKKIEFDKKINQSLKKIEESDKKKL-EAAAKK-$NH_2$ | 51 | Naked peptide |

Chol represented cholesteryl hemisuccinate, which indicated an ester that was formed by linking carboxyl group of cholesteryl hemisuccinate with the amino group of a lysine in a polypeptide

Table 1-6 Sequence design for polypeptide (such core sequence corresponding to Formula I)

| Compound corresponding to core sequence | Core sequence | SEQ ID NO: |
|---|---|---|
| Compound 1 | FDASIEEVNRRIEESLRRIEESDRRLEVNKKA | 52 |
| Compound 2 | DDEFNQKINEVNEKIEEILKKIEESDRKLEESDKKA | 53 |
| Compound 3 | DEFDKKKIEEVNKKIEESLKKIEESDKKLEEV | 54 |
| Compound 4 | FDASLLKIEEEKKKIEELLKLHNESDKKLEEV | 55 |
| Compound 5 | WDEFDKKIEEVNKKIEESLKKIEESDKKLEEV NKKL | 56 |
| Compound 9 | WDEFDKKIEEVNKKIEESLKKIEESDKKLEEV NKKL | 60 |
| Compound 10 | KIEEVNKKIEESLKKIEESDKKLEEVNKKL | 61 |
| Compound 11 | WDEFDKKIEEVNKKIEESLKKIEESDKKLEEV NKKL | 62 |
| Compound 12 | FDASIEEVNRRIEESLRRIEESDRRLEVNKKA | 63 |
| Compound 13 | WDEFDKKIEEVNKKIEESLKKIEESDKKL | 64 |
| Compound 14 | DEFDKKIEEVNKKIEESLKKIEESDKKLEEVN | 65 |
| Compound 15 | DEFDKKKIEEVNKKIEESLKKIEESDKKLEEV | 66 |
| Compound 16 | SDEFDKKIEEVNKKIEESLKKIEESDKKL | 67 |
| Compound 17 | DEFDKKIEEVNKKIEESLKKIEESDKKL | 68 |
| Compound 18 | DEFDKKKIEEVNKKIEESLKKIEESDKKLEEV | 69 |
| Compound 19 | FDASIEEVNRRIEESLRRIEESDRRL | 70 |
| Compound 20 | SDEFDKKIEEVNKKIEESLKKIEESDKKL | 71 |
| Compound 21 | DEFDKKIEEVNKKIEESLKKIEESDKKLEEV | 72 |
| Compound 22 | DEFDKKKIEEVNKKIEESLKKIEESDKKLEEV | 73 |

Table 1-7 Sequence design for polypeptide (such core sequence corresponding to Formula II)

| Compound corresponding to core sequence | Core sequence of compound | SEQ ID NO: |
|---|---|---|
| Compound 23 | YDEL VFDASQVN EKI NQSLEKI RKSDELLHNV | 74 |
| Compound 24 | FDFDEFDASISQVNEKINESLAFIRLAFIRKSDELL | 75 |
| Compound 25 | YDEL VFDASQVN EKI NQSLEKI RKSDELLHNV | 76 |

(continued)

| Compound corresponding to core sequence | Core sequence of compound | SE Q ID NO: |
|---|---|---|
| Compound 26 | FDEEISQVNEKINQSLAFIRKSDELLHNVNAGK STT | 77 |
| Compound 27 | WDEFDASISQVNEKINQSLEEIRKSDELLHNV NAGL | 78 |
| Compound 28 | DEFDASISQVNEKINQSLEEIRKSDELLHN | 79 |
| Compound 29 | LVKKIDEFDASISQVNEKINQSLEEIRKSDELLH NV | 80 |
| Compound 30 | YDEL VFDASQVN EKI NQSLEKI RKSDELLHNV | 81 |

Table 1-8 Sequence design for polypeptide (such core sequence corresponding to Formula III)

| Compound corresponding to core sequence | Core sequence of compound | SE Q ID NO: |
|---|---|---|
| Compound 40 | WDEFDKKIEEVNKKIEESLKKIEESDKKLEEV NA | 91 |
| Compound 41 | WDEFDKKIEEVNKKIEESLKKIEESDKKLEEV N | 92 |
| Compound 42 | FDASIEEVNRRIEESLRRIEESDRRLEEVNA | 93 |
| Compound 43 | WDEFDASIEEVNRRIEESLRRIEESDRRLEE V | 94 |
| Compound 44 | WDEFDKKIEEVNKKIEESLKKIEESDKKLHNV | 95 |

Table 1-9 Sequence design for polypeptide (such core sequence corresponding to Formula IV)

| Compound corresponding to core sequence | Core sequence of compound | SE Q |
|---|---|---|
| | | ID NO: |
| Compound 38 | WDEFDASISQVNEKINQSLEEIRKSDELLHNV NAL | 89 |
| Compound 39 | WDEFDASISQNEKINQSLEEIRKSDELLHNNA L | 90 |
| Compound 45 | WDEFDASISQVNEKINQSLEEIRKSDELLHN | 96 |

Table 1-10 Sequence design for polypeptide (core sequence)

| Compound corresponding to core sequence | Core sequence of compound | SE Q ID NO: |
|---|---|---|
| Compound 6 | ADAFRLEVNDASSKINESIEESLLSLEKLHNVN ATA | 57 |
| Compound 7 | FDAFIQEINVNEDQSLEQSDELLLELHLLHSLL H | 58 |

(continued)

| Compound corresponding to core sequence | Core sequence of compound | SE Q ID NO: |
|---|---|---|
| Compound 8 | FAEFNQKINQVNEKIEESLEEIRKSDEELHNVN ATT | 59 |
| Compound 31 | SDEQVNEKINQSLAFIRRIRKLLHN | 82 |
| Compound 32 | ILELVNKKIEQSLKFIEKSDKLLEN | 83 |
| Compound 33 | SLEQVNKKINQSLKVNKKSDKLLEN | 84 |
| Compound 34 | FDEEVNKKIEQSLKINQSLEEIRKS | 85 |
| Compound 35 | SISQVNEKINEIQSLEEKSDKLLKS | 86 |
| Compound 36 | VNKKIEEEKQSLKKQSDKIEESDEN | 87 |
| Compound 37 | FDEL VN KKI EKI EEVN KKSLKLLES | 88 |
| Compound 46 | FEVNRRRIEQSLEKSLESLEEEEHSDKKLHNE LH | 97 |
| Compound 47 | SLEQVNKINKKIDKIEESLKKIEESDKKSLEVN KKL | 98 |
| Compound 48 | SLEQVNKINEKINKISQSLKKIEESDKKSDEVN AGL | 99 |
| Compound 49 | SLEQVNKKIEQSLESLKKSDKINQSLEEVNKS DELL | 100 |
| Compound 50 | SLEQVNEKINQSLAFIRKSDEINQSDEEVNKS DELL | 101 |
| Compound 51 | SDELVNKKIEFDKKINQSLKKIEESDKKKL | 102 |

Table 1-11 Comparative example

| **Comparative example No.** | **Sequence structure of positive polypeptide** | Source of core sequence of positive polypeptide | SE Q ID NO: |
|---|---|---|---|
| Positive polypeptide 1 | Ac-SLEQVNKKIEQSLKFIEKSDKLLENGSGSGK-(Chol)-$NH_2$ | CN117186187B Applicant: Institute of Pathogenic Biology, Chinese Academy of Medical Sciences | 130 |

(continued)

| Comparative example No. | Sequence structure of positive polypeptide | Source of core sequence of positive polypeptide | SEQ ID NO: |
|---|---|---|---|
| Positive polypeptide 2 | Ac-IEQVNKKIEQSLKFIEKSDKLLENVNKGK-$NH_2$ | CN 111303245 B Applicant: Chengdu Aoda Bitechnololgy Co., Ltd. | 131 |
| Positive polypeptide 3 | Ac-IEEVNKKIEESLKKIEESDKKLEEVNKKK EAAAKK(Chol)-$NH_2$ | | 132 |
| Positive polypeptide 4 | Ac-IEQVNKKIEQSLKFIEKSDKLLENVNKGKC-EAAAKK(Chol)-$NH_2$ | | 133 |
| Positive polypeptide 5 | Ac-LEQVNKKIEQSLKFIEKSDKLLENVNKGKC-EAAAKK(Chol)-$NH_2$ | | 134 |
| Positive polypeptide 6 | Ac-VFPSDEFDASISQVNEKINQSLAAIRKSDELLHNV-$NH_2$ | US6623741B1, Applicant: Trimeris | 135 |
| Positive polypeptide 7 | Ac-FDASISQVNEKINQSLAFIRKSDELLHNVNAGKSTT - EAAAKK(Chol)-$NH_2$ | WO2010/089129A1 | 136 |

The meaning of Chol was as indicated previously.

**Embodiment 2: Synthesis of naked peptide**

**1. Chemical reagents required during preparation**

Chemical reagents used: for example, various Fmoc amino acids, N,N'-diisopropylcarbodiimide (DIC), 1-hydroxybenzotriazole (HOBt), N,N-dimethylformamide (DMF), piperidine (PIPE), ninhydrin, acetic anhydride (Ac2O), N,N-diisopropylethylamine (DIEA), trifluoroacetic acid (TFA), 1,2-ethanedithiol (EDT), Thioanisole (TA), Triisopropylsilane (TIPS), phenol, etc. all were purchased from main chemical reagent suppliers, and were not subjected to further purification before being used.

Protecting amino acid raw materials used during polypeptide synthesis included: Fmoc-Ala-OH, Fmoc-Arg(Pbf)-OH, Fmoc-Asp(OtBu)-OH, Fmoc-Asn(Trt)-OH, Fmoc-Glu(OtBu)-OH, Fmoc-Gln(Trt)-OH, Fmoc-Gly-OH, Fmoc-His(Trt)-OH, Fmoc-Ile-OH, Fmoc-Lys(Boc)-OH, Fmoc-Leu-OH, Fmoc-Phe-OH, Fmoc-Thr(tBu)-OH, Fmoc-Tyr(tBu)-OH, Fmoc-Ser(tBu)-OH, Fmoc-Val-OH, and Fmoc-Trp(Boc)-OH. The abbreviations had commonly known definitions: Fmoc was 9-fluorenylmethoxycarbonyl; Dde was 1-(4,4-dimethyl-2,6-dioxocyclohexylidene)ethyl; Boc was tertiary butyloxycarbonyl; tBu was tert butyl; OtBu was tert butoxy; and Trt was trityl; Pbf was (2,3-dihydro-2,2,4,6,7-pentamethylbenzofuran-5-yl) sulfonyl.

**2. Synthesis of peptide resin**

A Rink Amide MBHA resin was used as a carrier resin, and protecting amino acids corresponding to a polypeptide amino acid sequence were coupled through Fmoc deprotection and coupling reactions, so as to prepare a peptide resin.

### 2.1 Introduction of 1st protecting amino acid in main chain

0.3 mmol of a 1st protecting amino acid and 0.3 mmol of HOBt were taken and dissolved with a proper amount of a DMF; 0.3 mmol of DIC was additionally taken and slowly added to a protecting amino acid DMF solution with stirring; and the mixture was agitated for 5 min at a room temperature to obtain an activated protecting amino acid solution for subsequent use.

0.1 mmol of the Rink Amide MBHA resin (0.35 mmol/g*0.3 g) was taken, deprotection was performed for 20 min (twice) by using a 25% PIPE/ DMF solution (volume ratio), and a resin with Fmoc removed was obtained through washing and filtration.

The activated 1st protecting amino acid solution was added to the resin with Fmoc removed, a coupling reaction was performed for 60 min, and washing and filtration were performed to obtain a resin containing the 1st protecting amino acid.

### 2.2 Introduction of other protecting amino acids in main chain

The same method for introducing the 1st protecting amino acid was used to sequentially introduce other protecting amino acids corresponding to the polypeptide, so as to obtain a resin containing main chain amino acids.

Finally, 0.3 mmol $Ac_2O$+0.6 mmol DIEA were used to perform acetylation capping on an N-terminus to complete synthesis of the main chain.

The reactions were controlled through Kaiser Test after the above reactions in each step were performed. If an amino acid condensation reaction was incomplete, condensation was repeated once until a target peptide fragment required was obtained.

### 3. Preparation of crude product

The above peptide resin was taken, a lysis reagent (lysis reagent 15 mL/g resin) was added, after thorough mixing, the mixture was agitated at 30°C for 3 hours, a target polypeptide was cracked down from the resin, and a side-chain protecting group was removed. A filtrate of the reaction mixture was collected, the resin was washed for three times with a small amount of TFA/DCM, filtrates were combined, and then anhydrous ether was added for precipitation and centrifugation. A filter cake was washed and precipitated twice with cold anhydrous ether, and off-white powder was obtained through draining, which was a naked peptide crude product.

The composition and volume ratio of the lysis reagent were as follows: trifluoroacetic acid:1,2-ethane-dithiol:thioanisole: phenol:$H_2O$:triisopropylsilane = 68.5:10:10:5:3.5:1.

### 4. Preparation of pure product

The above naked peptide crude product was taken, stirred and dissolved by adding water/acetonitrile, and the mixture was centrifuged to remove insoluble for subsequent use.

Purification was performed by using reverse phase high-performance liquid chromatography: chromatographic column model: Agela C18; chromatographic column specifications: 10 μm, 100 Å, 50×250 mm; mobile phase: mobile phase A (0.05% TFA and 2% acetonitrile aqueous solution) and mobile phase B (90% acetonitrile/aqueous solution); mobile phase flow rate: 25 mL/min; UV detection wavelength: 220 nm; and elution mode: gradient elution. A crude product solution was loaded in the above chromatographic column, corresponding purification components were collected, and a solvent was directly removed through freeze-drying, so as to obtain a pure trifluoroacetate polypeptide in a fluffy state.

### 5. Characterization of pure product

The pure trifluoroacetate polypeptide was re-dissolved with water and acetonitrile, a large amount of anion exchange resin (acetate form) was added, and stirring was performed for 3 h. The ion exchange resin was filtered and washed with a water/acetonitrile mixture, and then the filtrates were combined and freeze-dried to obtain pure polypeptide acetate in a fluffy state (i.e., naked peptide in Table 1-1-Table 1-5 and Table 1-11). The chemical structure of the naked peptide was characterized by liquid chromatography-mass spectrometry; and the purity of each naked peptide, as well as the sequence structure, molecular weight, and purity of each naked peptide, were analyzed by an analytical high performance liquid chromatography (chromatographic column model: Agela C18; chromatographic column specifications: 4.6×250 mm, and flow rate: 1 mL/min).

**Conclusions:**

HPLC analysis confirmed that all synthesized naked peptides exhibited a purity greater than 95%, and mass spectrometry verified their correct molecular weights.

**Embodiment 3: Synthesis of lipopeptide**

**1. Chemical reagents required during preparation**

Chemical reagents used: hydrazine hydrate and cholesteryl hemisuccinate.
Protecting amino acid raw materials used during polypeptide synthesis included: Fmoc-Lys(Dde)-OH.

**2. Synthesis of overall sequence of naked peptide and linker arm sequences**

An adapter of a linker arm included: a peptide (SEQ ID NO:104) of an EAAAK sequence, and a peptide (SEQ ID NO:105) of a GSGSG sequence.
A lipophilic compound for modification included the cholesteryl hemisuccinate.

2.1. Synthesis of main chain

(1) Synthesis of peptide resin: a Rink Amide MBHA resin was used as a carrier resin, and protecting amino acids corresponding to a polypeptide amino acid sequence were coupled through Fmoc deprotection and coupling reactions, so as to prepare a peptide resin.

(2) Introduction of 1st protecting amino acid in main chain

0.3 mmol of a 1st protecting amino acid and 0.3 mmol of HOBt were taken and dissolved with an appropriate volume of DMF; 0.3 mmol of DIC was additionally taken and slowly added to a protecting amino acid DMF solution with stirring; and the mixture was agitated for 5 min at a room temperature to obtain an activated protecting amino acid solution for subsequent use.
0.1 mmol of the Rink Amide MBHA resin (0.35 mmol/g*0.3 g) was taken, deprotection was performed for 20 min (twice) by using a 25% PIPE/ DMF solution (volume ratio), and a resin with Fmoc removed was obtained through washing and filtration.
The activated 1st protecting amino acid solution was added to the resin with Fmoc removed, a coupling reaction was performed for 60 min, and washing and filtration were performed to obtain a resin containing the 1st protecting amino acid.

(3) Introduction of other protecting amino acids in main chain

The same method for introducing the 1st protecting amino acid was used to sequentially introduce other protecting amino acids corresponding to the polypeptide, so as to obtain a resin containing main chain amino acids. Finally, 0.3 mmol $Ac_2O$+0.6 mmol DIEA were used to perform acetylation capping on an N-terminus to complete synthesis of the main chain. The reactions were controlled through Kaiser Test after the above reactions in each step were performed. If an amino acid condensation reaction was incomplete, condensation was repeated once until a target peptide fragment required was obtained.

2.2 Side-chain introduction

(1) The resin was treated by using a 2% hydrazine hydrate/DMF solution (volume ratio) in a smallest possible volume to remove a Dde protecting group of a C-terminal lysine side chain (10 min, twice), and filtration and washing were performed to obtain a resin with Dde removed for subsequent use.

(2) Polypeptide C-terminal lysine lipophilic compound modification

Polypeptide C-terminal lysine cholesteryl hemisuccinate modification: 0.3 mmol of the cholesteryl hemisuccinate and 0.3 mmol of HOBt were taken and dissolved with a proper amount of a DMF; 0.3 mmol of DIC was additionally taken and slowly added to a solution containing the cholesteryl hemisuccinate and the HOBt; and the mixture was agitated for 5 min at a room temperature. The prepared solution containing the cholesteryl hemisuccinate, the HOBt, and the DIC was added to the resin with Dde removed obtained in step (1); and a coupling reaction was performed for 60 min, and filtration, washing, and drying were performed to obtain the peptide resin.

Other chemical reagents, amino acid raw materials, and operation steps were the same as Embodiment 1.

**Conclusions:**

Through HPLC result analysis, it was confirmed that the synthesized salt bridge polypeptides all showed a purity greater than 98%, and mass spectrometry determined that the molecular weight of the polypeptides was the same as a theoretical molecular weight.

**Embodiment 4: Anti-RSV activity detection for polypeptide**

In this embodiment, an RSV-A long virus strain was used as an infection virus in the present disclosure. The anti-RSV activity of the synthesized polypeptide was determined through CPE detection. It was determined that series activity was significantly superior to that of a new polypeptide of a polypeptide fusion inhibitor.

The inhibitory activity of the compounds of Table 1-1-Table 1-5, positive polypeptide in Table 1-11, and positive control T-118 against the RSV-A long virus strain was detected.

**1. Experimental material**

Human Epithelial Pharyngeal Carcinoma Cells (HEp-2) were from the American Type Culture Collection (ATCC), with a catalog number CCL-23. The cells were cultured in a DMEM culture solution in which 10% fetal bovine serum, 1% sodium pyruvate, 1% nonessential amino acids, 2 mM glutamine, 100 U/mL penicillin, and 100 μg/mL streptomycin were added. The RSV Along virus strain was from the ATCC, with a catalog number VR-26.

**2. Experimental method**

This study used a plaque reduction experiment to detect the in-vitro activity of a test sample against the RSV A long virus strain. The test sample and a control compound were tested at 8 concentrations in duplicated wells. The HEp-2 cells were digested with trypsin and then diluted to a concentration of 300,000 cells per milliliter by using an experimental culture solution. The diluted cells were added to a 96-well cell test board, with 100 μL per well, corresponding to 30000 cells per well. The cells were cultured overnight in a 37 °C incubator with 5% $CO_2$. On the second day, the test sample was diluted in an experimental culture medium at a fold ratio. The sample diluted at a fold ratio and an equal volume of virus (100 PFU per well) were well mixed and incubated at 37 °C in 5% $CO_2$ for 1 h. Then, the culture solution in the 96-well cell test board was discarded, the sample virus mixed solution (200 μL) was added to the 96-well cell culture plate, and incubated at 37 °C in 5% $CO_2$ for 2 hours. After 2 h, the sample virus mixed solution was discarded, and 200 μL of maintenance culture medium containing 0.8% CMC and the corresponding concentration of the test sample was added. A cell control (cells, without compound treatment or viral infection) and a viral control (cell infection virus, without compound treatment) were set. The cells were cultured for 1 day in the 37 °C incubator with 5% $CO_2$. One day after viral infection, the culture medium was discarded, the cells were fixed with 4% paraformaldehyde, and then permeabilization was performed with 0.5% Triton X-100. After the plate was washed with DPBS, an RSV-specific antibody (RSV antibody, diluted 1:3000) was added and incubated at 37 °C for 1 h. Then, a secondary antibody (donkey anti-goat IgG, diluted 1:500) and incubated at 37 °C for 1 h. The secondary antibody was discarded, a TrueBlue solution was added, dyeing was performed for 10 min; and the plate was washed with running water and then air-dried, and the number of spots in each well was counted by using a microplate imaging counter. Raw data was used for calculating the antiviral activity of the samples.

A cytotoxicity test and an antiviral test were performed in parallel. The HEp-2 cells were inoculated in the microplate at a density of 30,000 cells per well, and cultured overnight in the 37 °C incubator with 5% $CO_2$. On the next day, the diluted test sample was added. The cell control (cells, without compound treatment) and a culture solution control (culture solution only, without cell or compound treatment) were set. A final concentration of DMSO in the culture solution was 0.5%. The cells were cultured for 1 day in the 37 °C incubator with 5% $CO_2$. Cell viability was detected by using a cell viability detection kit CCK8.

The raw data for plaque counts and cell viability tests were used for analyzing the antiviral activity and cytotoxicity of the test samples, respectively. A calculation formula was shown as follows.

% inhibition rate = 100-(sample value-cell control average value)/(virus control average value-cell control average value)$\times$100

% cell survival rate = (sample value-culture medium control average value)/(cell control average value-culture medium control average value)$\times$100

Non-linear fitting analysis was performed on the inhibition rate of the sample by using GraphPad Prism (version 10), so as to calculate the $IC_{50}$ and $CC_{50}$ values for the sample.

$IC_{50}$: in an in-vitro antiviral activity experiment, $IC_{50}$ represented an inhibition ability indicator of a compound to be tested against the Hep-2 cells infected by the RSV A long virus strain. Specifically, it indicated that, when the compound to be tested reached a concentration, 50% infection of the Hep-2 cells by the RSV A long virus strain could be effectively inhibited, and this concentration was the $IC_{50}$.

$CC_{50}$: in the in-vitro antiviral activity experiment, $CC_{50}$ was used to measure the toxicity of the compound to be tested to the Hep-2 cells. To be precise, when the compound to be tested was at a specific concentration, 50% of the Hep-2 cells was caused to die, and this concentration was defined as the $CC_{50}$. A fitting formula was log(inhibitor) vs. response-Variable slope (four parameters).

$$\text{Safety index SI:SI}=IC_{50}/CC_{50}$$

Positive polypeptides 1 to 7 were used as comparative examples, and the anti-RSV virus activity of the polypeptide sample was detected. The cell fusion inhibition activity of the polypeptide sample and positive polypeptides were shown in Table 2 below.

Table 2 Cell fusion inhibition activity

| Compound name | $IC_{50}$/nM | $CC_{50}$/$\mu$M | Safety index (SI) |
|---|---|---|---|
| Compound 1 | 1361 | >8 | >5.88 |
| Compound 2 | >8000 | >8 | NA |
| Compound 3 | 4426 | >8 | >1.81 |
| Compound 4 | >8000 | >8 | NA |
| Compound 5 | 1123 | >8 | >7.12 |
| Compound 6 | >8000 | >8 | NA |
| Compound 7 | >8000 | >8 | NA |
| Compound 8 | 3769 | >8 | >2.12 |
| Compound 9 | 95.7 | >8 | >83.61 |
| Compound 10 | 18.1 | 1.4 | 79 |
| Compound 11 | 99.1 | >8 | >80.76 |
| Compound 12 | 28.4 | >8 | >281.29 |
| Compound 13 | 13.0 | 30.7 | 2358 |
| Compound 14 | 9.9 | 9.6 | 970 |
| Compound 15 | 10.4 | 8.9 | 856 |
| Compound 16 | 10.3 | 17.1 | 1655 |
| Compound 17 | 17.6 | 26.4 | 1497 |
| Compound 18 | 19.2 | 6.4 | 333 |
| Compound 19 | 16.2 | 8.3 | 514 |
| Compound 20 | 19.8 | 29.0 | 1470 |
| Compound 21 | 14.6 | 18.3 | 1259 |
| Compound 22 | 11.3 | 3.7 | 324 |

(continued)

| Compound name | $IC_{50}$/nM | $CC_{50}$/μM | Safety index (SI) |
|---|---|---|---|
| Compound 23 | 2334 | >8 | >3.43 |
| Compound 24 | 2648 | >8 | >3.02 |
| Compound 25 | 28.7 | 7.5 | 260 |
| Compound 26 | 12.8 | >100 | 7843 |
| Compound 27 | 25.7 | 18.4 | 717 |
| Compound 28 | 12.4 | 8.8 | 709 |
| Compound 29 | 19.3 | 15.0 | 778 |
| Compound 30 | 14.9 | 6.2 | 415 |
| Compound 31 | 2219 | >8 | >3.61 |
| Compound 32 | 295.6 | >8 | >27.06 |
| Compound 33 | 5037 | >8 | >1.59 |
| Compound 34 | >2000 | 1.5 | NA |
| Compound 35 | 5529 | 5.1 | 1 |
| Compound 36 | 6823 | >8 | 1 |
| Compound 37 | 5226 | 4.0 | 1 |
| Compound 38 | 14.6 | 10.89 | 745.38 |
| Compound 39 | 112.7 | >100 | >887.31 |
| Compound 40 | 11.7 | 13.05 | 1119.21 |
| Compound 41 | 14.8 | 16.98 | 1144.98 |
| Compound 42 | 11.8 | 3.71 | 314.06 |
| Compound 43 | 18.3 | 12.25 | 671.23 |
| Compound 44 | 12.4 | 26.46 | 2137.32 |
| Compound 45 | 12.2 | 9.94 | 815.34 |
| Compound 46 | >8000 | >8 | NA |
| Compound 47 | >8000 | >8 | NA |
| Compound 48 | >8000 | >8 | NA |
| Compound 49 | >8000 | >8 | NA |
| Compound 50 | 15.1 | 32.6 | 2160 |
| Compound 51 | 9.9 | 3.2 | 322 |
| Positive polypeptide 1 | 150 | 3.69 | 24.84 |
| Positive polypeptide 2 | >300 | >10 | NA |
| Positive polypeptide 3 | >300 | >10 | NA |
| Positive polypeptide 4 | 190 | 4.62 | 24.14 |
| Positive polypeptide 5 | >300 | 3.72 | NA |
| Positive polypeptide 6 | >300 | >10 | NA |
| Positive polypeptide 7 | >300 | >10 | NA |

The safety index (SI) was CC50/IC50; and NA: could not be calculated, not available.

**Conclusions**

1. The anti-RSV virus activity of the designed salt bridge naked peptide sample was very high.

2. The salt bridge naked peptide sample prepared in the present disclosure was high in safety, had a safety index SI > 1, and thus might serve as a safe and effective alternative drug against the RSV virus.

**Embodiment 5: Pharmacological effect of polypeptide in mouse challenge protection ability study**

**1. Experimental material**

Female BALB/c mice were from Shanghai Yishang Biotechnology Co., Ltd. HEp-2 were from the ATCC, with a catalog number CCL-23. The cells were cultured in a DMEM medium solution in which 10% (v/v) fetal bovine serum and 1% penicillin and streptomycin were added. The RSV A2 virus strain was from the ATCC, with a catalog number VR-1540.

**2. Experimental method**

Eight-week-old female BALB/c mice were grouped based on body weight (n=6). All animals were intranasally challenged with RSV on Day 0 at a challenge dosage of $1\times10^6$ pfu (20μL) per animal. Administration began 1 h after Day 0 challenge; the polypeptide sample, positive control, and solvent control were administered via tracheal nebulization once daily for 5 consecutive days (see Table 3 below for administration dosage and administration frequency). Lung tissue was collected from all animals 2 h after the final administration on Day 4; left lung tissue was rapidly frozen in a virus protection solution with a 10-fold volume and stored below -70 °C; and a viral titer was detected by using a plaque method.

For analysis, the lung RSV A2 virus was determined by performing a tissue homogenate viral load plaque method. In short, on the day prior to sample detection, the HEp-2 cells were inoculated into a 12-well plate (spreading density being 0.3×106 cells/mL, with 1000 μL of cell suspension per well) according to a standard HEp-2 cell culture operation procedure. On the day of incubation, the supernatant was taken from the tissue sample that was homogenized using a tissue homogenizer (maintaining a low-temperature environment), and the supernatant was taken after centrifugation. The tissue homogenate sample was diluted with serum-free DMEM, and inoculated into a 12-well plate at a final volume of 400 μL. After 2 h of incubation, the homogenate supernatant was discarded, and 1.5 mL of a covering layer (cell culture medium containing 1% soft agar) was added to each well and cultured in the incubator for approximately 72 h. The covering layer was discarded, the cells were fixed, blocking was performed by using a blocking solution, dyeing was performed with HRP-RSVG, development and air drying were terminated with pure water. Colored spots on the well plate were counted by using a fluorescent (enzyme-linked) immunoassay spot analyzer, and the viral titer was expressed as a plaque-forming unit per gram of tissue (PFU/g). The viral titer was calculated as an arithmetic mean±standard error of all animals in a group.

Table 3 RSV A2 plaque detection results and statistics of each group

| Group | Test substance | Administration route | Administration dosage, administration frequency | Mean (PFU/g lung) |
|---|---|---|---|---|
| G1 | Solvent | Tracheal nebulization | 0 mg/kg, QD | 15917 |
| G2 | Compound 13 | Tracheal nebulization | 1 mg/kg, QD | 5380 |
| G3 | Compound 14 | Tracheal nebulization | 1 mg/kg, QD | 4820 |
| G4 | Compound 17 | Tracheal nebulization | 1 mg/kg, QD | 7885 |
| G5 | Compound 19 | Tracheal nebulization | 1 mg/kg, QD | 8650 |
| G6 | Compound 20 | Tracheal nebulization | 1 mg/kg, QD | 8928 |
| G7 | Compound 21 | Tracheal nebulization | 1 mg/kg, QD | 7512 |
| G8 | Compound 26 | Tracheal nebulization | 1 mg/kg, QD | 5015 |
| G9 | Compound 27 | Tracheal nebulization | 1 mg/kg, QD | 9458 |
| G10 | Compound 28 | Tracheal nebulization | 1 mg/kg, QD | 8425 |
| G11 | Compound 40 | Tracheal nebulization | 1 mg/kg, QD | 5678 |

(continued)

| Group | Test substance | Administration route | Administration dosage, administration frequency | Mean (PFU/g lung) |
|---|---|---|---|---|
| G12 | Positive control 1 | Tracheal nebulization | 1 mg/kg, QD | 13568 |
| G13 | Positive control 4 | Tracheal nebulization | 1 mg/kg, QD | 14525 |
| Note: "QD" stands for once daily. | | | | |

One hour after RSV A2 challenge in mice, compounds 13, 14, 17, 19, 20, 21, 26, 27, 28, and 40 were administered via tracheal nebulization once daily for 5 consecutive days; and 2 h after the final administration, lung tissue was collected from all groups for viral plaque detection, and results were shown in Table 3.

Pharmacological effect experiments of the compounds in mice indicated that the RSV compounds 13, 14, 17, 19, 20, 21, 26, 27, 28, and 40 could significantly reduce the viral titer in lung tissue.

**Embodiment 6: Pharmacological effect of polypeptide in cotton rats challenge protection ability study**

**1. Experimental material**

Female cotton rats were from Shanghai Yishang Biotechnology Co., Ltd. The RSV A2 virus strain was from the ATCC, with a catalog number VR-1540. RT-qPCR primer+probe primer were supplied by Qingke Biotechnology Co., Ltd, with batch number TSP20241227-025-00187. The RT-qPCR standard was provided by Vazyme, with batch number 20241008.

**2. Experimental method**

Female cotton rats (6-8 weeks old) were grouped based on body weight (n=5). Operations were performed on cotton rats as shown in Table 4 below. All animals were intranasally challenged with RSV on Day 0 at a challenge dosage of $1\times10^{5.5}$ pfu (50μL) per animal. Administration began 1 h after Day 0 challenge; the polypeptide sample, positive control, and solvent control were administered via tracheal nebulization once daily for 5 consecutive days. Lung tissue was collected from all animals 2 h after the final administration on Day 4. Left lung tissue (including partial trachea) was rapidly frozen in a virus protection solution with a 10-fold volume and stored below -70 °C; and the viral titer was detected with RT-qPCR.

Steps for RT-qPCR:

First, sample homogenization and lung tissue RNA extraction were performed, and the supernatant was taken from the tissue sample that was homogenized using a tissue homogenizer (maintaining a low-temperature environment). The approximately 100 μL of the homogenate supernatant was taken, and total tissue RNA was extracted by using a VeZol reagent-chloroform-water phase-magnetic bead method, and dissolved in RNase-free water. The RT-qPCR reaction system was prepared as follows: a one-step RT-qPCR reaction system was prepared by using an RNA template, a probe, a primer pair, an RT-qPCR buffer, and the RNase-free water; each sample was tested in duplicated wells, with each well containing 20 μL of the one-step RT-qPCR reaction system; a quantitative standard curve well (RSV A2 N gene full-length plasmid), a positive quality control well, and a negative quality control well were arranged at the same time. Then, amplification programs were set according to reverse transcription, pre-denaturation, and cyclic amplification, and amplification data was collected by using a real-time fluorescence quantification PCR instrument, and a mean CT value for duplicated wells was calculated. The CT value from the test results for each sample was substituted into a quantitative standard curve to calculate a copy number. A calculation formula for RSV viral load (copy number/gram of lung tissue) was: sample viral load (copy number/gram of lung tissue) = copy number/relative tissue input (g).

Experimental results were shown in Table 4.

Table 4 Pharmacological effect test results of compounds in cotton rats

| Group | Test substance and administrati on | Administrati on route | Administrati on dosage, administrati on frequency | Nucleic acid load RT-qPCR detection results |
|---|---|---|---|---|
| | | | | Log10 mean (copy number/gram of lung tissue) |
| G1 | Solvent | Tracheal nebulization | 0 mg/kg, QD | 10.17 |
| G2 | Compound 13 | Tracheal nebulization | 1 mg/kg, QD | 7.82 |
| G3 | Compound 14 | Tracheal nebulization | 1 mg/kg, QD | 7.68 |
| G4 | Compound 17 | Tracheal nebulization | 1 mg/kg, QD | 8.42 |
| G5 | Compound 19 | Tracheal nebulization | 1 mg/kg, QD | 8.35 |
| G6 | Compound 20 | Tracheal nebulization | 1 mg/kg, QD | 8.55 |
| G7 | Compound 21 | Tracheal nebulization | 1 mg/kg, QD | 8.19 |
| G8 | Compound 26 | Tracheal nebulization | 1 mg/kg, QD | 7.78 |
| G9 | Compound 27 | Tracheal nebulization | 1 mg/kg, QD | 8.86 |
| G10 | Compound 28 | Tracheal nebulization | 1 mg/kg, QD | 7.76 |
| G11 | Compound 40 | Tracheal nebulization | 1 mg/kg, QD | 7.71 |
| G12 | Positive control 1 | Tracheal nebulization | 1 mg/kg, QD | 9.98 |
| G13 | Positive control 4 | Tracheal nebulization | 1 mg/kg, QD | 10.25 |
| Note: "QD" stands for once daily. | | | | |

One hour after RSV A2 challenge in cotton rats, compounds 13, 14, 17, 19, 20, 21, 26, 27, 28, and 40 were administered via tracheal nebulization once daily for 5 consecutive days; and 2 h after the final administration, lung tissue was collected from all groups for viral titer detection, and results were shown in Table 4.

Pharmacological effect experiments of the compounds in cotton rats indicated that the RSV compounds 13, 14, 17, 19, 20, 21, 26, 27, 28, and 40 could significantly reduce the viral titer in lung tissue.

**Embodiment 7:** Antiviral activity detection

hMPV and hPIV were two viruses having a typical Class I fusion protein-F protein. An F protein precursor F0 was cracked into F1 and F2 subunits. HRN and HRC regions of the F1 subunit interacted with each other to form a 6-helix bundle (6-HB) during conformational change, so as to drive fusion between a viral membrane and a host cell membrane. By using a Surface Plasmon Resonance (SPR) technology, hMPV-N46 (127-172) and hPIV-N51 (139-189) from the HRN region were respectively coupled on a CM5 chip. When the compound flowed through a chip surface, changes in an SPR signal were monitored in real time, binding and dissociation processes were recorded, and a binding rate constant (ka) and a dissociation rate constant (kd) were further obtained through kinetic analysis, thereby calculating an equilibrium dissociation constant (KD = kd/ka).

**1. Experimental material**

A CM5 chip with a catalog number 29149603, an amino coupling reagent kit with a catalog number BR100050, HBS-EP + Buffer (10×), sodium acetate (pH 4.0), and a 10 mM glycine-HCl buffer (pH 2.0) were all purchased from GE Health.

**2. Experimental method**

CM5 chip coupling: Channels 2 and 4 of the CM5 chip were activated for 7 min by using a freshly-prepared mixture of 50 mM Hydroxy succinimide (NHS) and 200 mM 1-(3-Dimethylaminopropyl)-3-ethylcarbodiimide(EDC) according to 1:1; and then, hMPV-N46 or hPIV-N51 was diluted and dissolved with sodium acetate with 4.0 pH to a concentration of 30 μM, with a flow rate of 10 μl/min and time of 600 s. An experimental buffer was HBS-EP + buffer (1×). Finally, the chip was blocked with 1 M ethanolamine. The instrument was set to a temperature of 25 °C.

Affinity detection: the buffer used in the experiment was HBS-EP+ buffer (1×), and a multi-cycle dynamic mode

was used. Channels 1 and 3 of the chip served as blank reference channels; polypeptides to be detected sequentially flowed through Channels 2 and 4 at concentrations of 0 μM, 0.04 μM, 0.12 μM, 0.37 μM, 1.11 μM, 3.33 μM, 10 μM, and 30 μM; the flow rate was 30 μl/min, binding was performed for 180 s, and dissociation was performed for 120 s. Finally, the chip was regenerated by injecting 10 mM glycine (pH 2.0). The instrument was set to a temperature of 25 °C.

Data analysis: data was analyzed by using BioCore T200 analysis software (Version 3.2.1); a reference channel and a zero-concentration background signal were subtracted, and a 1:1 binding model was selected for analysis.

Affinity detection results for the polypeptide compounds were shown in Table 5 below.

Table 5 Polypeptide-virus affinity experiment data

| **Compound name** | **hMPV affinity KD (M)** | **hPIV affinity KD (M)** |
|---|---|---|
| Compound 1 | NA | NA |
| Compound 2 | NA | NA |
| Compound 3 | NA | NA |
| Compound 4 | NA | NA |
| Compound 5 | NA | NA |
| Compound 6 | NA | NA |
| Compound 7 | NA | NA |
| Compound 8 | NA | NA |
| Compound 9 | NA | NA |
| Compound 10 | NA | NA |
| Compound 11 | NA | NA |
| Compound 12 | NA | NA |
| Compound 13 | $3.207\times10^{-6}$ | $2.282\times10^{-6}$ |
| Compound 14 | $4.779\times10^{-8}$ | $4.951\times10^{-7}$ |
| Compound 15 | $3.813\times10^{-8}$ | $1.386\times10^{-6}$ |
| Compound 16 | $3.063\times10^{-6}$ | $5.397\times10^{-6}$ |
| Compound 17 | $3.998\times10^{-8}$ | $3.138\times10^{-7}$ |
| Compound 18 | $9.143\times10^{-6}$ | $8.611\times10^{-6}$ |
| Compound 19 | NA | NA |
| Compound 20 | $1.908\times10^{-5}$ | $6.798\times10^{-6}$ |
| Compound 21 | NA | NA |
| Compound 22 | $1.443\times10^{-5}$ | $2.018\times10^{-5}$ |
| Compound 23 | NA | NA |
| Compound 24 | NA | NA |
| Compound 25 | $2.108\times10^{-5}$ | $7.455\times10^{-5}$ |
| Compound 26 | $2.151\times10^{-6}$ | $2.575\times10^{-6}$ |
| Compound 27 | $2.924\times10^{-6}$ | $8.116\times10^{-7}$ |
| Compound 28 | $2.087\times10^{-7}$ | $9.83\times10^{-4}$ |
| Compound 29 | $4.825\times10^{-8}$ | $1.73\times10^{-6}$ |
| Compound 30 | $1.866\times10^{-7}$ | $1.017\times10^{-6}$ |
| Compound 31 | NA | NA |
| Compound 32 | NA | NA |
| Compound 33 | NA | NA |
| Compound 34 | NA | NA |

(continued)

| Compound name | hMPV affinity KD (M) | hPIV affinity KD (M) |
|---|---|---|
| Compound 35 | NA | NA |
| Compound 36 | NA | NA |
| Compound 37 | NA | NA |
| Compound 38 | $3.924\times10^{-7}$ | $2.447\times10^{-5}$ |
| Compound 39 | NA | NA |
| Compound 40 | $2.473\times10^{-6}$ | $2.267\times10^{-6}$ |
| Compound 41 | $4.728\times10^{-6}$ | $4.921\times10^{-7}$ |
| Compound 42 | $5.327\times10^{-7}$ | $4.852\times10^{-7}$ |
| Compound 43 | $7.135\times10^{-5}$ | $5.721\times10^{-6}$ |
| Compound 44 | $1.495\times10^{-7}$ | $6.862\times10^{-6}$ |
| Compound 45 | $1.065\times10^{-5}$ | $1.185\times10^{-4}$ |
| Compound 46 | NA | NA |
| Compound 47 | NA | NA |
| Compound 48 | NA | NA |
| Compound 49 | NA | NA |
| Compound 50 | NA | NA |
| Compound 51 | $1.440\times10^{-8}$ | $5.142\times10^{-8}$ |
| NA: Not detected; and M: molar concentration mol/L. | | |

**Conclusions:** biological activity was a key quality attribute reflecting the efficacy of biological products, thereby requiring particular attention to research and validation of the biological activity. The binding activity of a receptor might be determined through SPR.

1. The KD value of the designed polypeptide compound and the hMPV was between μM and nM, belonging to moderate binding affinity, thereby indicating that the polypeptide compound had certain antiviral activity against the hMPV.

2. The KD value of the designed polypeptide compound and the hPIV was between μM and nM, belonging to moderate binding affinity, thereby indicating that the polypeptide compound had certain antiviral activity against the hPIV.

**Embodiment 8: Anti-hMPV viral activity detection-plaque reduction experiment**

**1. Laboratory supplies**

African green monkey kidney cells (Vero) were sourced from the ATCC, with catalog number CCL-81. The cells were cultured in a DMEM culture solution in which 10% fetal bovine serum, 1% non-essential amino acids, 1% sodium pyruvate, 1% L-glutamine, 100 U/ml penicillin, and 100 μg/ml streptomycin were added.
A hMPV plaque experiment culture medium consisted of an Opti-MEM culture solution added with 1% non-essential amino acids, 100 U/mL penicillin, and 100 μg/mL streptomycin.
hMPV virus strains A2 and B1 were constructed by WuXi AppTec.

**2. Experimental method**

This study used a plaque reduction experiment to detect the in-vitro activity of a test sample against the hMPV virus strains A2 and B1.
The test sample and an experimental system were tested at 8 concentrations in duplicated wells. The Vero cells

were digested with trypsin and then diluted to a concentration of 400,000 cells per milliliter by using a cell culture solution containing 2% serum. The diluted cells were added to a 96-well cell test board, with 100 μL per well, corresponding to 40000 cells per well. The cells were cultured overnight in a 37 °C incubator with 5% $CO_2$.

On the second day, the test sample was diluted in an experimental culture medium at a fold ratio. The sample diluted at a fold ratio and an equal volume of virus (approximately 200 PFU per well) were well mixed and incubated at 33 °C in 5% $CO_2$ for 1 h. Then, the culture solution in the 96-well cell test board was discarded, the sample virus mixed solution (100 μL) was added to the 96-well cell culture plate, and incubated at 37 °C in 5% $CO_2$ for 2 hours. After 2 h, the sample virus mixed solution was discarded, 200 μL of an experiment culture medium containing the test sample with a corresponding concentration was added, and the culture solution contained 0.8% CMC and 5 μg/ml trypsin. A final concentration of DMSO in the culture solution was 0.5%. A system control antibody was directly added to the experiment culture solution containing 0.8% CMC and 5 μg/ml trypsin. A cell control (cells, without compound treatment or viral infection) and a viral control (cell infection virus, without compound treatment) were set. The cells were cultured for 1 day in the 33 °C incubator with 5% $CO_2$.

The cells were fixed with 4% paraformaldehyde, and then permeabilization was performed with 0.5% Triton X-100. After the plate was washed with DPBS, a hMPV specific antibody (1:2000 dilution) was added and incubated at 37 °C for 1.5 h. Then, a secondary antibody (1:1000 dilution) was added and incubated at 37 °C for 1 hour. The secondary antibody was discarded, a TrueBlue solution was added, dyeing was performed for approximately 10 minutes; and the plate was washed with running water and then air-dried, and the number of spots in each well was counted by using a microplate imaging counter.

Raw data was used for analyzing the antiviral activity of the test samples. A calculation formula was shown as follows.

% inhibition rate = 100-(sample value-cell control average value)/(virus control average value-cell control average value)×100

Non-linear fitting analysis was performed on the inhibition rate of the sample by using GraphPad Prism (version 10), so as to calculate an $EC_{50}$ value for the sample. A fitting formula was log(inhibitor) vs. response-Variable slope (four parameters).

Table 6 Activity experiment data of compound against hMPV A2 and B1 viruses

| NO. | Compound serial number | **$EC_{50}$ (nM)** | |
|---|---|---|---|
| | | hMPV A2 | hMPV B1 |
| 1 | Compound 13 | 11.74 | 6.18 |
| 2 | Compound 14 | 18.9 | 5.36 |
| 3 | Compound 15 | 17.95 | 5.18 |
| 4 | Compound 16 | 7.35 | 4.5 |
| 5 | Compound 17 | 8.78 | 3.61 |
| 6 | Compound 18 | 35.91 | 79.67 |
| 7 | Compound 20 | 7.47 | 4.27 |
| 8 | Compound 21 | 11.15 | 4.92 |
| 9 | Compound 22 | 88.43 | 18.02 |
| 10 | Compound 25 | 86.39 | 38.06 |
| 11 | Compound 26 | 103.1 | 2.85 |
| 12 | Compound 27 | 78.32 | 10.51 |
| 13 | Compound 28 | 58.57 | 13.82 |
| 14 | Compound 29 | 41.65 | 11.06 |
| 15 | Compound 30 | 94.77 | 51.33 |
| 16 | Compound 38 | 127.5 | 16.62 |
| 17 | Compound 40 | 13.85 | 7.47 |

(continued)

| NO. | Compound serial number | $EC_{50}$ (nM) | |
|---|---|---|---|
| | | hMPV A2 | hMPV B1 |
| 18 | Compound 41 | 11.66 | 9.46 |
| 19 | Compound 42 | 26.14 | 10.11 |
| 20 | Compound 43 | 16.82 | 8.01 |
| 21 | Compound 44 | 14.59 | 6.6 |
| 22 | Compound 45 | 200.5 | 13.06 |
| 23 | Compound 50 | 13.27 | 5.95 |
| 24 | Compound 51 | 18.76 | 3.82 |

**Embodiment 9: Anti-hPIV3 viral activity detection-plaque reduction experiment**

**1. Laboratory supplies**

Rhesus monkey kidney cells (LLC-MK2) were sourced from the ATCC, with a catalog number CCL-7.1. The cells were cultured in a DMEM culture solution in which 10% fetal bovine serum, 1% non-essential amino acids, 1% sodium pyruvate, 1% L-glutamine, 100 U/ml penicillin, and 100 μg/ml streptomycin were added.

An HPIV-3 plaque experiment culture medium consisted of a DMEM culture solution added with 2% fetal bovine serum, 1% non-essential amino acids, 1% sodium pyruvate, 1% L-glutamine, 100 U/ml penicillin, and 100 μg/ml streptomycin.

The hPIV Type 3 (hPIV3 C243) was sourced from the ATCC, with a catalog number VR-93.

**2. Experimental method**

This study used the plaque reduction experiment to detect the in-vitro activity of the test sample against the hPIV3 C243 virus strain, and Human Anti-HPIV3 Monoclonal Antibody, clone PIA174 was an experimental system control. The test sample and a system experimental antibody were tested at 8 concentrations in duplicated wells.

The LLC-MK2 cells were digested with trypsin and then diluted to a concentration of 400,000 cells per milliliter by using a cell culture solution containing 2% serum. The diluted cells were added to a 96-well cell test board, with 100 μL per well, corresponding to 40,000 cells per well. The cells were cultured overnight in a 37 °C incubator with 5% $CO_2$.

On the next day, the sample diluted at a fold ratio and an equal volume of virus (approximately 200 PFU per well) were well mixed and incubated at 37 oC in 5% $CO_2$ for 1 hour. Then, the culture solution in the 96-well cell test board was discarded, the sample virus mixed solution (100 μL) was added to the 96-well cell culture plate, and incubated at 37 °C in 5% $CO_2$ for 2 hours. After 2 h, the sample virus mixed solution was discarded, 200 μL of an experiment culture medium containing the test sample with a corresponding concentration was added, and the culture solution contained 0.8% CMC. A final concentration of DMSO in the culture solution was 0.5%. A control antibody was directly added to the experiment culture solution containing 0.8% CMC. A cell control (cells, without compound treatment or viral infection) and a viral control (cell infection virus, without compound treatment) were set. The cells were cultured for 1 day in the 37 °C incubator with 5% $CO_2$.

The cells were fixed with 4% paraformaldehyde, and then permeabilization was performed with 0.5% Triton X-100. After the plate was washed with DPBS, a hPIV specific antibody (1:2000 dilution) was added and incubated at 37 °C for 1.5 h. Then, a secondary antibody (1:500 dilution) was added and incubated at 37 °C for 1 hour. The secondary antibody was discarded, a TrueBlue solution was added, dyeing was performed for approximately 10 minutes; and the plate was washed with running water and then air-dried, and the number of spots in each well was counted by using a microplate imaging counter.

Raw data was used for calculating the antiviral activity of the samples, and a calculation formula was shown as follows.

% inhibition rate = 100-(sample value-cell control average value)/(virus control average value-cell control average value)×100

Non-linear fitting analysis was performed on the inhibition rate of the sample by using GraphPad Prism (version

10), so as to calculate an $EC_{50}$ value for the sample. A fitting formula was log(inhibitor) vs. response-Variable slope (four parameters).

Table 7 Activity experiment data of compound against hPIV3 virus

| NO. | Compound serial number | $EC_{50}$ (nM) |
|---|---|---|
| 1 | Compound 13 | 35.56 |
| 2 | Compound 14 | 31.41 |
| 3 | Compound 15 | 20.7 |
| 4 | Compound 16 | 59.02 |
| 5 | Compound 17 | 52.59 |
| 6 | Compound 18 | 50.48 |
| 7 | Compound 20 | 59.46 |
| 8 | Compound 21 | 21.33 |
| 9 | Compound 22 | 65.41 |
| 10 | Compound 25 | 29.43 |
| 11 | Compound 26 | 1.35 |
| 12 | Compound 27 | 27.18 |
| 13 | Compound 28 | 54.42 |
| 14 | Compound 29 | 26.31 |
| 15 | Compound 30 | 48.21 |
| 16 | Compound 38 | 26.37 |
| 17 | Compound 40 | 22.22 |
| 18 | Compound 41 | 71.13 |
| 19 | Compound 42 | 18.95 |
| 20 | Compound 43 | 16.09 |
| 21 | Compound 44 | 27.93 |
| 22 | Compound 45 | 36.34 |
| 23 | Compound 50 | 34.78 |
| 24 | Compound 51 | 18.79 |

Although specific implementations of the present disclosure have been described in detail, those skilled in the art will understand that various modifications and substitutions may be made to those details based on all the teachings disclosed herein, and such changes are within the scope of the present disclosure. The full scope of the present disclosure is given by the appended claims and equivalents thereof.

## Claims

**1.** A compound, and a pharmaceutically acceptable salt or derivative thereof, wherein the compound, the pharmaceutically acceptable salt or derivative thereof comprise polypeptides that are selected from one or more of the following sequences shown in Formula I, Formula II, Formula III, Formula IV, SEQ ID NO:57-59, SEQ ID NO:82-88, and SEQ ID NO:97-102:

Formula I (SEQ ID NO:122):

$$X_1\text{-}X_2\text{-}X_3\text{-}X_4\text{-}X_5\text{-}X_6\text{-}X_7\text{-}X_8\text{-}X_9\text{-I-}X_{11}\text{-E-}X_{13}\text{-}X_{14}\text{-}X_{15}\text{-}X_{16}\text{-IEE-}X_{20}\text{-L-}X_{22}\text{-}X_{23}\text{-}X_{24}\text{-}X_{25}\text{-ESD-}X_{29}\text{-}X_{30}\text{-L-}X_{32}\text{-}X_{33}\text{-}X_{34}\text{-}X_{35}\text{-}X_{36}\text{-}X_{37}\text{-}X_{38},$$

wherein $X_1$ is D or absent; $X_2$ is E or absent; $X_3$ is W, F, D, S, or absent; $X_4$ is D or absent; $X_5$ is E, A, K, or absent; $X_6$ is F, S, or absent; $X_7$ is D, N, L, or absent; $X_8$ is K, Q, L, or absent; $X_9$ is K or absent; $X_{11}$ is E or N; $X_{13}$ is V or E; $X_{14}$ is N or K; $X_{15}$ is K, R, or E; $X_{16}$ is K or R; $X_{20}$ is S, I, or L; $X_{22}$ is K or R; $X_{23}$ is K or R; $X_{24}$ is I or H; $X_{25}$ is E or N; $X_{29}$ is K or R; $X_{30}$ is K or R; $X_{32}$ is E or absent; $X_{33}$ is E, V, or absent; $X_{34}$ is V, N, S, or absent; $X_{35}$ is N, K, D, or absent; $X_{36}$ is K or absent; $X_{37}$ is K, A, or absent; and $X_{38}$ is L, A, or absent;
Formula II (SEQ ID NO:123):

$$X'_1\text{-}X'_2\text{-}X'_3\text{-}X'_4\text{-}X'_5\text{-}X'_6\text{-E-}X'_8\text{-}X'_9\text{-}X'_{10}\text{-}X'_{11}\text{-}X'_{12}\text{-}X'_{13}\text{-}X'_{14}\text{-SQVNEKIN-}X'_{23}\text{-SL-}X'_{26}\text{-}X'_{27}\text{-IR-}X'_{30}\text{-}X'_{31}\text{-}X'_{32}\text{-}X'_{33}\text{-}X'_{34}\text{-KSDELL-}X'_{41}\text{-}X'_{42}\text{-}X'_{43}\text{-}X'_{44}\text{-}X'_{45}\text{-}X'_{46}\text{-}X'_{47}\text{-}X'_{48}\text{-}X'_{49}\text{-}X'_{50},$$

wherein $X'_1$ is F, W, D, L, or Y; $X'_2$ is V or absent; $X'_3$ is K or absent; $X'_4$ is D, K, or absent; $X'_5$ is F, I, or absent; $X'_6$ is D or absent; $X'_8$ is L or absent; $X'_9$ is V or absent; $X'_{10}$ is F or absent; $X'_{11}$ is E or D; $X'_{12}$ is A or I; $X'_{13}$ is S or absent; $X'_{14}$ is I or absent; $X'_{23}$ is E or Q; $X'_{26}$ is A or E; $X'_{27}$ is F, E, or K; $X'_{30}$ is L or absent; $X'_{31}$ is A or absent; $X'_{32}$ is F or absent; $X'_{33}$ is I or absent; $X'_{34}$ is R or absent; $X'_{41}$ is H or absent; $X'_{42}$ is N or absent; $X'_{43}$ is V or absent; $X'_{44}$ is N or absent; $X'_{45}$ is A or absent; $X'_{46}$ is G or absent; $X'_{47}$ is K, L, or absent; $X'_{48}$ is S or absent; $X'_{49}$ is T or absent; and $X'_{50}$ is T or absent;
Formula III (SEQ ID NO:124):

$$X''_1\text{-}X''_2\text{-}X''_3\text{-}X''_4\text{-D-}X''_6\text{-}X''_7\text{-IEEVN-}X''_{13}\text{-}X''_{14}\text{-IEESL-}X''_{20}\text{-}X''_{21}\text{-IEESD-}X''_{27}\text{-}X''_{28}\text{-L-}X''_{30}\text{-}X''_{31}\text{-V-}X''_{33}\text{-}X''_{34},$$

wherein $X''_1$ is W or F; $X''_2$ is D or absent; $X''_3$ is E or absent; $X''_4$ is F or absent; $X''_6$ is K or A; $X''_7$ is K or S; $X''_{13}$ is K or R; $X''_{14}$ is K or R; $X''_{20}$ is K or R; $X''_{21}$ is K or R; $X''_{27}$ is K or R; $X''_{28}$ is K or R; $X''_{30}$ is E or H; $X''_{31}$ is E or N; $X''_{33}$ is N or absent; $X''_{34}$ is A or absent; and
Formula IV (SEQ ID NO:125):
WDEFDASISQ-$X'''_{11}$-NEKINQSLEEIRKSDELLHN-$X'''_{32}$-$X'''_{33}$-$X'''_{34}$-$X'''_{35}$, wherein $X'''_{11}$ is V or absent; $X'''_{32}$ is V, N, or absent; $X'''_{33}$ is N, A, or absent; $X'''_{34}$ is A, L, or absent; and $X'''_{35}$ is L or absent.

2. The compound, the pharmaceutically acceptable salt or derivative thereof according to claim 1, wherein the compound, the pharmaceutically acceptable salt or derivative thereof comprise polypeptides that are selected from one or more of sequences shown in SEQ ID NO:52-102.

3. The compound, the pharmaceutically acceptable salt or derivative thereof according to claim 1, wherein the structure of the compound is $R_1$-XX-$R_2R_3$, wherein XX is selected from amino acid sequences shown in Formula I, Formula II, Formula III, Formula IV, SEQ ID NO:57-59, SEQ ID NO:82-88, and SEQ ID NO:97-102 according to claim 1; $R_1$ is an amino-terminal protecting group; $R_2$ is absent or an arbitrarily-substituted linker arm; and $R_3$ is a carboxyl-terminal protecting group.

4. The compound, the pharmaceutically acceptable salt or derivative thereof according to claim 3, wherein the compound, the pharmaceutically acceptable salt or derivative thereof comprise polypeptides that are selected from one or more of the following sequences shown in Formula I-1, Formula II-1, Formula III-1, Formula IV-1, and SEQ ID NO:106-121:

Formula I-1 (SEQ ID NO:126):

$$R_1\text{-}X_1\text{-}X_2\text{-}X_3\text{-}X_4\text{-}X_5\text{-}X_6\text{-}X_7\text{-}X_8\text{-}X_9\text{-I-}X_{11}\text{-E-}X_{13}\text{-}X_{14}\text{-}X_{15}\text{-}X_{16}\text{-IEE-}X_{20}\text{-L-}X_{22}\text{-}X_{23}\text{-}X_{24}\text{-}X_{25}\text{-ESD-}X_{29}\text{-}X_{30}\text{-L-}X_{32}\text{-}X_{33}\text{-}X_{34}\text{-}X_{35}\text{-}X_{36}\text{-}X_{37}\text{-}X_{38}\text{-}R_2\text{-}R_3;$$

Formula II-1 (SEQ ID NO:127):

$R_1$-X'$_1$-X'$_2$-X'$_3$-X'$_4$-X'$_5$-X'$_6$-E-X'$_8$-X'$_9$-X'$_{10}$-X'$_{11}$-X'$_{12}$-X'$_{13}$-X'$_{14}$-SQVNEKIN-X'$_{23}$-SL-X'$_{26}$-X'$_{27}$-IR-X'$_{30}$-X'$_{31}$-X'$_{32}$-X'$_{33}$-X'$_{34}$-KSDELL-X'$_{41}$-X'$_{42}$-X'$_{43}$-X'$_{44}$-X'$_{45}$-X'$_{46}$-X'$_{47}$-X'$_{48}$-X'$_{49}$-X'$_{50}$-$R_2$-$R_3$;

Formula III-1 (SEQ ID NO:128):

$R_1$-X''$_1$-X''$_2$-X''$_3$-X''$_4$-D-X''$_6$-X''$_7$-IEEVN-X''$_{13}$-X''$_{14}$-IEESL-X''$_{20}$-X''$_{21}$-IEESD-X''$_{27}$-X''$_{28}$-L-X''$_{30}$-X''$_{31}$-V-X''$_{33}$-X''$_{34}$-$R_2$-$R_3$;

and
Formula IV-1 (SEQ ID NO:129):
$R_1$-WDEFDASISQ-X'''$_{11}$-NEKINQSLEEIRKSDELLHN-X'''$_{32}$-X'''$_{33}$-X'''$_{34}$-X'''$_{35}$-$R_2$-$R_3$.

**5.** The compound, the pharmaceutically acceptable salt or derivative thereof according to claim 3 or 4, wherein

the $R_1$ is acetyl; and/or the $R_2$ is -$R_4$-$R_5$($R_6$)-, wherein $R_4$ is a peptide fragment, $R_5$ is lysine, cysteine, 2,3-diaminopropionic acid, ornithine, 2,4-diamino-butyric acid, or 2,7-diaminoheptanoic acid, and $R_6$ is a lipophilic compound group linked to $R_5$; and/or the $R_3$ is - $NH_2$;
preferably, an amino acid sequence of the $R_4$ is $(EAAAK)_m$ or $(GSGSG)_m$, wherein m is a natural number from 0 to 5; and/or
the lipophilic compound group is selected from one or more of cholesterol, cholesteryl hemisuccinate, 2-cholesterol acetic acid, 2-cholesterol propionic acid, 3-cholesterol propionic acid, 2-cholesterol butyric acid, 2-cholesterol isobutyric acid, 3-cholesterol butyric acid, 3-cholesterol isobutyric acid, 4-cholesterol butyric acid, 2-cholesterol valeric acid, 2-cholesterol isovaleric acid, 3-cholesterol valeric acid, 5-cholesterol valeric acid, 2-cholesterol hexanoic acid, 6-cholesterol hexanoic acid, 2-cholesterol heptanoic acid, 7-cholesterol heptanoic acid, 2-cholesterol octanoic acid, 8-cholesterol octanoic acid, bromoacetate cholesterol ester, cholesteryl chloroformate, palmitic acid, stearic acid, fatty acid containing 3-20 carbon atoms, and binary fatty acid containing 3-20 carbon atoms; and the lipophilic compound group is able to act with a cell membrane or viral envelope to improve the binding of the peptide fragment and the cell membrane or viral envelope.

**6.** The compound, the pharmaceutically acceptable salt or derivative thereof according to claim 1, wherein the compound, the pharmaceutically acceptable salt or derivative thereof comprise polypeptides that are selected from one or more of sequences shown in SEQ **ID** NO:1-51;
optionally, the derivative is a solvate, a chelate, or a non-covalent complex.

**7.** The compound, the pharmaceutically acceptable salt or derivative thereof according to any one of claims 1 to 4 for use as a polypeptide membrane fusion inhibitor against *Paramyxoviridae* and/or *Pneumoviridae* viruses.

**8.** An isolated nucleic acid molecule, encoding the compound, the pharmaceutically acceptable salt or derivative thereof according to any one of claims 1 to 5.

**9.** A recombinant vector, comprising the nucleic acid molecule according to claim 8.

**10.** A recombinant cell, comprising the nucleic acid molecule according to claim 8 or the recombinant vector according to claim 9.

**11.** A pharmaceutical composition, comprising the compound, the pharmaceutically acceptable salt or derivative thereof according to any one of claims 1 to 6, the nucleic acid molecule according to claim 8, the recombinant vector according to claim 9, or the recombinant cell according to claim 10;
optionally, further comprising a pharmaceutically acceptable carrier or excipient.

**12.** A formulation product, comprising the pharmaceutical composition according to claim 11;
preferably, a dosage form of the formulation product comprises one of the following groups: an oral dosage form, an injectable dosage form, an inhalation dosage form, and a freeze-dried dosage form, preferably, the inhalation dosage form, and the freeze-dried dosage form, a subcutaneous injection dosage form, or an intramuscular injection dosage form;optionally, an administration route of the formulation product is selected from one of the following: oral administration, injection, mucosal administration, transdermal administration, and nebulization, preferably pulmon-

ary nebulization, subcutaneous injection, or intramuscular injection.

**13.** A method for preparing the compound, the pharmaceutically acceptable salt or derivative thereof according to any one of claims 1 to 6, wherein the preparation method comprises the following steps:

using a carrier resin to couple protecting amino acids corresponding to a polypeptide amino acid sequence through deprotection and coupling reactions, so as to prepare a salt-bridge naked peptide;
and preferably, the method further comprises one or more of the following steps:

(1) performing a lipophilic compound modification, preferably, a cholesteryl hemisuccinate modification;
(2) performing N-terminal acetylation capping; and
(3) performing C-terminal amidation capping.

**14.** The compound, the pharmaceutically acceptable salt or derivative thereof according to any one of claims 1 to 6, the pharmaceutical composition according to claim 12, or the formulation product according to claim 12 for use in the prevention and/or treatment of diseases caused by *Paramyxoviridae* and/or *Pneumoviridae* viruses;
preferably, the *Paramyxoviridae* virus is selected from viruses of the genus *Respirovirus, Rubulavirus, Morbillivirus,* and *Henipavirus;* the *Pneumoviridae* virus is selected from viruses of the genus *Metapneumovirus* and *Orthopneumovirus;*
preferably, the virus of the genus *Respirovirus* is a human parainfluenza virus; the virus of the genuse *Rubulavirus* is *Mumpsvirus;* the virus of the genus *Morbillivirus* is a measles virus; the virus of the genus *Henipavirus* is selected from *Nipahvirus* and *Hendravirus;* the virus of the genus *Metapneumovirus* is human metapneumovirus; and the virus of the genus *Orthopneumovirus* is Respiratory Syncytial Virus (RSV).

**15.** A method for inhibiting *Paramyxoviridae* and/or *Pneumoviridae* viruses, wherein the method comprises applying, to a sample, the compound, the pharmaceutically acceptable salt or derivative thereof according to any one of claims 1 to 6, the pharmaceutical composition according to claim 11, or the formulation product according to claim 12.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 117186187 B
- CN 111303245 B
- US 6623741 B1
- WO 2010089129 A1